(19) Europäisches Patentamt
European Patent Office
Office européen des brevets

(11) Veröffentlichungsnummer: **0 194 548**
**A2**

(12) # EUROPÄISCHE PATENTANMELDUNG

(21) Anmeldenummer: **86102773.8**

(22) Anmeldetag: **03.03.86**

(51) Int. Cl.4: **C07C 143/79**, A61K 31/18, A61K 31/215, A61K 31/38, A61K 31/44, A61K 31/50, C07D 237/04, C07D 213/71, C07D 333/34, C07D 237/14, C07D 401/12

(30) Priorität: **12.03.85 DE 3508692**
**27.11.85 DE 3541854**

(43) Veröffentlichungstag der Anmeldung:
**17.09.86 Patentblatt 86/38**

(84) Benannte Vertragsstaaten:
**AT BE CH DE FR GB IT LI LU NL SE**

(71) Anmelder: **Dr. Karl Thomae GmbH**
**Postfach 1755**
**D-7950 Biberach (Riss)(DE)**

(72) Erfinder: **Nickl, Josef, Dr. Dipl.-Chem.**
**Silcherstrasse 8**
**D-7950 Biberach 1(DE)**
Erfinder: **Haarmann, Walter, Dr.**
**Schlierholzweg 27**
**D-7950 Biberach 1(DE)**
Erfinder: **Ballhause, Helmut**
**Fohrenweg 6**
**D-7950 Biberach 1(DE)**
Erfinder: **Weisenberger, Johannes, Dr. Dipl.-Chem.**
**Haydnweg 5**
**D-7950 Biberach 1(DE)**
Erfinder: **Heckel, Armin, Dr. Dipl.-Chem.**
**Lamparterweg 1**
**D-7950 Biberach 1(DE)**
Erfinder: **Müller, Erich, Dr. Dipl.-Chem.**
**Talfeldstrasse 34**
**D-7950 Biberach 1(DE)**
Erfinder: **Narr, Berthold, Dr. Dipl.-Chem.**
**Obere Au 5**
**D-7950 Biberach 1(DE)**

(54) **Neue Sulfonylaminoäthylverbindungen, diese Verbindungen enthaltende Arzneimittel und Verfahren zu ihrer Herstellung.**

(57) Die Erfindung betrifft neue Sulfonylaminoäthylverbindungen der allgemeinen Formel

$$R_1-SO_2\overset{R_2}{\overset{|}{N}}-CH_2-A-B-E \quad ,(I)$$

in der

$R_1$ eine gegebenenfalls durch eine Methylgruppe oder ein Halogenatom mono-oder disubstituierte Phenylgruppe, wobei die Substituenten gleich oder verschieden sein können, eine gegebenenfalls durch ein Halogenatom substituierte Biphenylylgruppe, eine Naphthyl-, Pyridyl-oder Thienylgruppe,

$R_2$ ein Wasserstoffatom oder eine Methylgruppe,

A eine Methylen-oder Methylenoxygruppe, wobei das Sauerstoffatom mit dem benachbarten Rest B verknüpft ist,

EP 0 194 548 A2

B eine Phenylen-oder Naphthylengruppe und

E eine über eine geradkettige oder verzweigte Alkylen-oder Alkenylengruppe gebundene Hydroxycarbonyl-oder Alkoxycarbonylgruppe, wobei jeweils eine Methylengruppe der vorstehend erwähnten Alkylen-oder Alkenylenreste, welche mit dem Rest B verknüpft sein muß, durch eine Hydroxymethylen-oder Carbonylgruppe ersetzt ist, oder eine gegebenenfalls im Kohlenstoffgerüst durch 1 oder 2 Alkylgruppen substituierte 4,5-Dihydro-pyridazin-3-on-6-yl-gruppe bedeuten, und deren Salze mit anorganischen oder organischen Basen, falls E eine Hydroxycarbonylgruppe enthält.

Die neuen Verbindungen weisen wertvolle pharmakologische Eigenschaften auf, insbesondere antithrombotische Wirkungen, außerdem stellen diese Thromboxanantagonisten dar, und lassen sich nach an und für sich bekannten Verfahren herstellen.

Neue Sulfonylaminoäthylverbindungen, diese Verbindungen enthaltende Arzneimittel und Verfahren zu ihrer Herstellung

In der japanischen Offenlegungsschrift 124.279/78 - (siehe C.A. 90, 168.635t (1979)) werden unter anderem die Verbindungen der Formel

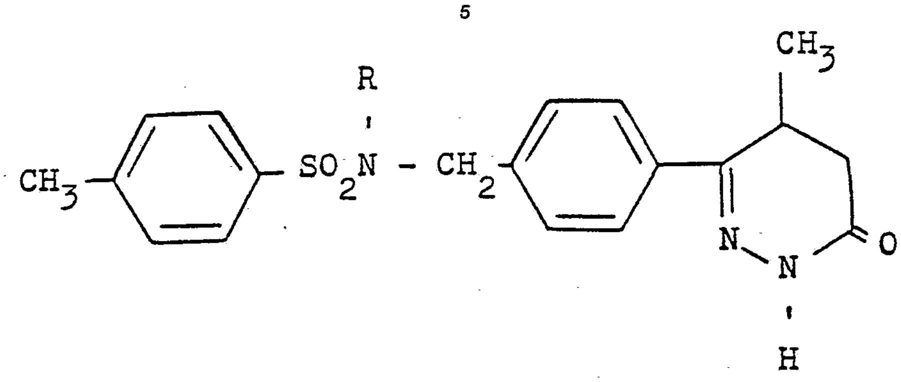

in der

R ein Wasserstoffatom oder eine Methylgruppe bedeutet, beschrieben, welche wertvolle pharmakologische Eigenschaften aufweisen, unter anderem eine Hemmwirkung auf die Plättchenaggregation und antithrombotische Wirkungen.

Außerdem werden in DE-A-3.000.377 4-(Benzolsulfonamidoäthyl)-benzoesäuren, Benzolsulfonamidoäthyl-essigsäuren, -propionsäuren und -acrylsäuren sowie deren Ester beschrieben, welche eine lipidsenkende und eine thrombozytenaggregationshemmende Wirkung aufweisen.

Es wurde nun gefunden, daß die neuen Verbindungen der allgemeinen Formel

$$R_1-SO_2\overset{R_2}{\overset{|}{N}}-CH_2-A-B-E \qquad ,(I)$$

und deren physiologisch verträgliche Salze mit anorganischen oder organischen Basen, falls E eine Hydroxycarbonylgruppe enthält, überlegene pharmakologische Eigenschaften aufweisen, insbesondere antithrombotische Wirkungen, außerdem stellen die neuen Verbindungen Thromboxanantagonisten dar.

Gegenstand der vorliegenden Erfindung sind somit die neuen Verbindungen der obigen allgemeinen Formel I, deren Additionssalze mit anorganischen oder organischen Basen, insbesondere für die pharmazeutische Verwendung deren physiologisch verträgliche Additionssalze, diese Verbindungen enthaltende Arzneimittel und Verfahren zu ihrer Herstellung.

In der obigen allgemeinen Formel bedeutet

$R_1$ eine gegebenenfalls durch eine Methylgruppe oder ein Halogenatom mono-oder disubstituierte Phenylgruppe, wobei die Substituenten gleich oder verschieden sein können, eine gegebenenfalls durch ein Halogenatom substituierte Biphenylylgruppe, eine Naphthyl-, Pyridyl-oder Thienylgruppe,

$R_2$ ein Wasserstoffatom oder eine Methylgruppe,

A eine Methylen-oder Methylenoxygruppe, wobei das Sauerstoffatom mit dem benachbarten Rest B verknüpft ist,

B eine Phenylen-oder Naphthylengruppe und

E eine über eine geradkettige oder verzweigte Alkylengruppe mit 2 bis 5 Kohlenstoffatomen oder über eine Alkenylengruppe mit 3 bis 5 Kohlenstoffatomen gebundene Hydroxycarbonyl-oder Alkoxycarbonylgruppe, wobei jeweils eine Methylengruppe der vorstehend erwähnten Alkylen- oder Alkenylenreste, welche mit dem Rest B verknüpft sein muß, durch eine Hydroxymethylen-oder Carbonylgruppe ersetzt ist und der Alkoxyteil, welcher in 2-oder 3-Stellung durch eine Alkoxygruppe mit 1 bis 3 Kohlenstoffatomen substituiert sein kann, 1 bis 3 Kohlenstoffatomen enthalten kann, oder eine gegebenenfalls im Kohlenstoffgerüst durch 1 oder 2 Alkylgruppen mit insgesamt 1 bis 3 Kohlenstoffatomen substituierte 4,5-Dihydro-pyridazin-3-on-6-yl-oder Pyridazin-3-on-6-yl-gruppe.

Für die bei der Definition der Reste $R_1$, B und E eingangs erwähnten Bedeutungen kommt beispielsweise

für $R_1$ die der Phenyl-, 2-Methylphenyl-, 3-Methylphenyl-, 4-Methylphenyl-, 2-Fluorphenyl,-, 3-Fluorphenyl-, 4-Fluorphenyl-, 2-Chlorphenyl-, 3-Chlorphenyl-, 4-Chlorphenyl-, 2-Bromphenyl-, 4-Bromphenyl-, 3,4-Dimethylphenyl-, 2,4-Difluorphenyl-, 2,4-Dichlorphenyl-, 2,4-Dibrom-phenyl-, Methyl-chlorphenyl-, Naphth-1-yl-, Naphth-2-yl-, 4-Biphenylyl-, 2'-Fluor-4-biphenylyl-, 2'-Chlor-4-biphenylyl-, 2'-Brom-4-biphenylyl-, 3'-Fluor-4-biphenylyl-,

4'-Fluor-4-biphenylyl-, 4'-Chlor-4-biphenylyl-, 2-Fluor-4-biphenylyl-, 3-Chlor-4-biphenylyl-, 2-Brom-4-biphenylyl-, Pyrid-2-yl-, Pyrid-3-yl-, Pyrid-4-yl-, Thien-2-yl-oder Thien-3-yl-gruppe,

für B die 1,4-Phenylen-, 1,3-Phenylen-oder 1,4-Naphthylengruppe und

für E der 2-Hydroxycarbonyl-äthanon-(1)-yl-, 3-Hydroxycarbonyl-n-propanon-(1)-yl-, 4-Hydroxycarbonyl-n-butanon-(1)-yl-, 5-Hydroxycarbonyl-n-pentanon-(1)-yl-, 2-Hydroxycarbonyl-2-methyl-äthanon-(1)-yl-, 3-Hydroxycarbonyl-2-methyl-n-propanon-(1)-yl-, 3-Hydroxycarbonyl-3-methyl-n-propanon-(1)-yl-, 4-Hydroxycarbonyl-2-methyl-n-butanon-(1)-yl-, 4-Hydroxycarbonyl-3-methyl-n-butanon-(1)-yl-, 4-Hydroxycarbonyl-4-methyl-n-butanon-(1)-yl-, 2-Hydroxycarbonyl-2-äthyl-äthanon-(1)-yl-, 2-Hydroxycarbonyl-2-n-propyl-äthanon-(1)-yl-, 2-Hydroxycarbonyl-2-äthyl-n-propanon-(1)-yl-, 3-Hydroxycarbonyl-3-äthyl-n-propanon-(1)-yl-, 3-Hydroxycarbonyl-2-methyl-n-propanon-(1)-yl-, 3-Hydroxycarbonyl-3-methyl-n-propanon-(1)-yl-, 4-Hydroxycarbonyl-n-buten-2-on-(1)-yl-, 5-Hydroxycarbonyl-n-penten-2-on-(1)-yl-, 2-Hydroxycarbonyl-1-hydroxy-äthyl-, 3-Hydroxycarbonyl-1-hydroxy-n-propyl-, 4-Hydroxycarbonyl-1-hydroxy-n-butyl-, 5-Hydroxycarbony-1-hydroxy-n-pentyl-, 2-Hydroxycarbonyl-2-methyl-1-hydroxy-äthyl-, 2-Hydroxycarbonyl-2-äthyl-1-hydroxy-äthyl-, 2-Hydroxycarbonyl-2-isopropyl-1-hydroxy-äthyl-, 3-Hydroxycarbonyl-2-methyl-1-hydroxy-n-propyl-, 3-Hydroxycarbonyl-2-äthyl-1-hydroxy-n-propyl-, 3-Hydroxycarbonyl-3-methyl-1-hydroxy-n-propyl-, 3-Hydroxycarbonyl-3-äthyl-1-hydroxy-n-propyl-, 4-Hydroxycarbonyl-2-methyl-1-hydroxy-n-butyl-, 4-Hydroxycarbonyl-3-methyl-1-hydroxy-n-butyl-, 4-Hydroxycarbonyl-4-methyl-1-hydroxy-n-butyl-, 2-Methoxycarbonyl-äthanon-(1)-yl-, 2-(2-Methoxyäthoxycarbonyl)-äthanon-(1)-yl-, 3-Methoxycarbonyl-n-propanon-(1)-yl-, 3-Äthoxycarbonyl-n-propanon-(1)-yl-, 3-(2-Äthoxyäthoxycarbonyl)-n-propanon(1)-yl-, 3-(3-Methoxy-n-propoxycarbonyl)-n-propanon-(1)-yl-, 3-n-Propoxycarbonyl-n-propanon-(1)-yl-, 4-Äthoxycarbonyl-n-butanon-(1)-yl-, 5-Äthoxycarbonyl-n-pentanon-(1)-yl-, 2-Äthoxycarbonyl-2-methyl-äthanon-(1)-yl-, 3-Äthoxycarbonyl-2-methyl-n-propanon-(1)-yl-, 3-Äthoxycarbonyl-3-methyl-n-propanon-(1)-yl-, 4-Äthoxycarbonyl-2-methyl-n-butanon-(1)-yl-, 4-Äthoxycarbonyl-3-methyl-n-butanon-(1)-yl-, 4-Äthoxycarbonyl-4-methyl-n-butanon-(1)-yl-, 2-Äthoxycarbonyl-2-äthyl-äthanon-(1)-yl-, 2-Äthoxycarbonyl-2-n-propyl-äthanon-(1)-yl-, 3-Äthoxycarbonyl-2-äthyl-n-propanon-(1)-yl-, 3-Äthoxycarbonyl-3-äthyl-n-propanon-(1)-yl-, 3-Äthoxycarbonyl-n-propanon-(1)-yl-, 3-Äthoxycarbonyl-2-methyl-n-propanon-(1)-yl-, 3-Äthoxycarbonyl-3-methyl-n-propanon-(1)-yl-, 4-Äthoxycarbonyl-n-buten-2-on-(1)-yl-, 5-Äthoxycarbonyl-n-penten-2-on-(1)-yl-, Äth oxycarbonylhydroxymethyl-, 2-Äthoxycarbonyl-1-hydroxy-äthyl-, 3-Äthoxycarbonyl-1-hydroxy-n-propyl-, 4-Äthoxycarbonyl-1-hydroxy-n-butyl-, 5-Äthoxycarbonyl-1-hydroxy-n-pentyl-, 2-Äthoxycarbonyl-2-methyl-1-hydroxy-äthyl-, 2-Äthoxycarbonyl-2-äthyl-1-hydroxy-äthyl-, 2-Äthoxycarbonyl-2-isopropyl-1-hydroxy-äthyl-, 3-Äthoxycarbonyl-2-methyl-1-hydroxy-n-propyl-, 3-Äthoxycarbonyl-2-äthyl-1-hydroxy-n-propyl-, 3-Äthoxycarbonyl-3-methyl-1-hydroxy-n-propyl-, 3-Äthoxycarbonyl-3-äthyl-1-hydroxy-n-propyl-, 4-Äthoxycarbonyl-2-methyl-1-hydroxy-n-butyl-, 4-Äthoxycarbonyl-3-methyl-1-hydroxy-n-butyl-, 4-Äthoxycarbonyl-4-methyl-1-hydroxy-n-butyl-, 3-(2-Methoxyäthoxycarbonyl)-n-propanon(1)-yl-, 3-(2-Äthoxyäthoxycarbonyl)-n-propanon-(1)-yl-, 3-(2-Isopropoxyäthoxycarbonyl)-n-propanon-(1)-yl-, 3-(3-Methoxy-n-propoxycarbonyl)-n-propanon-(1)-yl-, 3-(3-n-Propoxy-n-propoxycarbonyl)-n-propyl-(1)-yl-, 4,5-Dihydro-pyridazin-3-on-6-yl-, 4,5-Dihydro-5-methyl-pyridazin-3-on-6-yl-, 4,5-Dihydro-5-äthyl-pyridazin-3-on-6-yl-, 4,5-Dihydro-5-n-propyl-pyridazin-3-on-6-yl-, 4,5-Dihydro-5,5-dimethyl-pyridazin-3-on-6-yl-, Pyridazin-3-on-6-yl-, 5-Methyl-pyridazin-3-on-6-yl-, 5-Äthyl-pyridazin-3-on-6-yl-, 5-n-Propyl-pyridazin-3-on-6-yl-oder 5-Isopropyl-pyridazin-3-on-6-yl-gruppe in Betracht.

Bevorzugte Verbindungen der obigen allgemeinen Formel I sind diejenigen, in denen

$R_1$ eine gegebenenfalls durch eine Methylgruppe, durch ein Fluor-, Chlor-oder Bromatom substituierte Phenylgruppe, eine gegebenenfalls durch ein Fluor-oder Chloratom substituierte 4-Biphenylgruppe, eine Naphthyl-, Pyridyl-oder Thienylgruppe,

$R_2$ ein Wasserstoffatom oder eine Methylgruppe,

A eine Methylen-oder Methylenoxygruppe, wobei das Sauerstoffatom mit dem benachbarten Rest B verknüpft ist,

B eine 1,4-Phenylen-oder 1,4-Naphthylengruppe und

E eine über eine geradkettige oder verzweigte Alkylengruppe mit 2 bis 4 Kohlenstoffatomen oder über eine geradkettige oder verzweigte Alkenylengruppe mit 3 oder 4 Kohlenstoffatomen gebundene Hydroxycarbonyl-oder Alkoxycarbonylgruppe mit insgesamt 2 oder 3 Kohlenstoffatomen, wobei jeweils eine Methylengruppe der vorstehend erwähnten Alkylen-oder Alkenylengruppen, welche mit dem Rest B verknüpft sein muß, durch eine Hydroxymethylen-oder Carbonylgruppe ersetzt ist und eine Äthoxycarbonylgruppe in 2-Stellung durch eine Methoxygruppe substituiert sein kann, wobei jedoch E keine gegebenenfalls in α-Stellung durch eine Methyl-oder Äthylgruppe substituierte Hydroxycarbonylmethylencarbonylgruppe darstellen kann, oder eine gegebenenfalls in 5-Stellung durch eine Methylgruppe substituierte 4,5-Dihydro-pyridazin-3-on-6-yl-oder Pyridazin-3-on-6-yl-gruppe bedeuten.

Besonders bevorzugte Verbindungen der obigen allgemeinen Formel I sind jedoch diejenigen, in denen

$R_1$ eine gegebenenfalls durch ein Chloratom oder eine Methylgruppe substituierte Phenylgruppe,

$R_2$ ein Wasserstoffatom,

A eine Methylen-oder Methylenoxygruppe,

B eine 1,4-Phenylengruppe und

E eine über eine n-Propylengruppe gebundene Hydroxycarbonyl-oder Alkoxycarbonylgruppe mit insgesamt 2 oder 3 Kohlenstoffatomen, wobei jeweils eine Methylengruppe der vorstehend erwähnten Propylengruppe, welche mit dem Rest B verknüpft sein muß, durch eine Hydroxymethylen-oder Carbonylgruppe ersetzt ist und eine Äthoxycarbonylgruppe in 2-Stellung durch eine Methoxygruppe substituiert sein kann, oder eine gegebenenfalls in 5-Stellung durch eine Methylgruppe substituierte 4,5-Dihydro-pyridazin-3-on-6-yl-gruppe bedeuten.

Erfindungsgemäß erhält man die neuen Verbindungen nach folgenden Verfahren:

a.) Acylierung einer Verbindung der allgemeinen Formel

$$\begin{matrix} R_2 \\ | \\ H - N - CH_2 - A - B - E \end{matrix} \quad ,(II)$$

in der

$R_2$, A, B und E wie eingangs definiert sind, wobei eine Hydroxygruppe im Rest E durch eine hydrolytisch oder hydrogenolytisch abspaltbare Schutzgruppe wie eine Alkoxy-oder Benzyloxygruppe geschützt sein kann, mit einem Phenylsulfonsäurederivat der allgemeinen Formel

$R_1$ $-SO_2X$ ,(III)

in der

$R_1$ wie eingangs definiert ist und

X eine nucleophile Austrittsgruppe wie ein Halogenatom oder eine Alkoxygruppe, z.B. ein Chlor-oder Bromatom, eine Methoxy-oder Äthoxygruppe, darstellt und gegebenenfalls anschließende Abspaltung eines verwendeten Schutzrestes.

Die Umsetzung wird vorzugsweise in einem Lösungsmittel wie Methanol, Äthanol, Wasser/Methanol, Dioxan oder Tetrahydrofuran gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50°C, vorzugsweise jedoch bei Raumtemperatur, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Dei Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b.) Zur Herstellung von Verbindungen der allgemeinen Formel I, in der E eine über eine Alkylen-oder Alkenylgrupe gebundene Hydroxycarbonylgruppe darstellt:

Abspaltung eines Schutzrestes von einer Verbindung der allgemeinen Formel

$$\begin{matrix} R_2 \\ | \\ R_1-SO_2N-CH_2-A-B-E_1 \end{matrix} \quad ,(IV)$$

in der

$R_1$, $R_2$, A und B wie eingangs definiert sind und $E_1$ die für E eingangs erwähnten Bedeutungen besitzt, wobei jedoch die Carboxygruppe durch einen hydrolytisch, thermolytisch oder hydrogenolytisch abspaltbare Schutzgruppe geschützt ist oder ein funktionelles Derivat der Carboxygruppe darstellt und/oder der Rest $E_1$ eine durch einen Schutzrest geschützte Hydroxygruppe enthält.

Als hydrolysierbare Gruppen kommen beispielsweise funktionelle Derivate der Carboxygruppe wie deren unsubstituierte oder substituierte Amid, Ester, Thioester, Orthoester, Iminoäther, Amidine oder Anhydride, die Nitrilgruppe, Äthergruppen wie die Methoxy-oder Benzyloxygruppe oder Lactone und

als thermolytisch abspaltbare Gruppen beispielsweise Ester mit tertiären Alkoholen, z.B. der tert. Butylester, und als hydrogenolytisch abspaltbare Gruppen beispielsweise Aralkylgruppen, z.B. die Benzylgruppe, in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen ziwschen -10 und 120°C, z.B. bei Temperaturen zwischen Raumtemperatur und der Siedetemperatur des Reaktionsgemisches, durchgeführt.

Enthält beispielsweise eine Verbindung der allgemeinen Formel IV eine Nitril-oder Aminocarbonylgruppe, so können diese Gruppen vorzugsweise mittels 100%iger Phosphorsäure bei Temperaturen zwischen 100 und 180°C, vorzugsweise bei Temperauturen zwischen 120 und 160°C, oder auch mit einem Nitrit, z.B. Natriumnitrit, in Gegenwart

einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Enthält beispielsweise eine Verbindung der allgemeinen Formel IV die tert.Butoxycarbonylgruppe, so kann die tert.-Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfon-säure, Schwefelsäure, Phosphorsäure oder Polyphosphorsäure vorzugsweise bei der Siedetemperatur des verwendeten Lö-sungsmittels, z.B. bei Temperaturen zwischen 40 und 100°C, abgespalten werden.

Enthält beispielsweise eine Verbindung der allgemeinen Formel IV die Benzyloxy-oder Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur und einem Wasserstoffdruck von 1 bis 5 bar abgespalten werden. Bei der Hydrogenolyse kann gleichzeitig eine halogenhaltige Verbindung enthalogeniert und eine vorhandende Doppelbindung aufhydriert werden.

c.) Zur Herstellung von Verbindungen der allgemeinen Formel I, in der E benachbart zum Rest B eine Carbonylgruppe enthält:

Acylierung einer Verbindung der allgemeinen Formel

$$R_1-SO_2\overset{\displaystyle R_2}{\overset{|}{N}}-CH_2-A-B-H \qquad ,(V)$$

in der

$R_1$, $R_2$, A und B wie eingangs definiert sind, mit einer Verbindung der allgemeinen Formel

Y -$E_2$ ,(VI)

in der

$E_2$ die für E eingangs erwähnten Bedeutungen besitzt, wobei jedoch E benachbart zu Y eine Carbonylgruppe enthalten muß und gleichzeitig eine gegebenenfalls vorhandene Hydroxycarbonylgruppe durch einen hydrolytisch oder hydrogenolytisch abspaltbaren Schutzrest wie eine Alkoxy-oder Benzylgruppe geschützt sein kann, und

Y eine nucleophile Austrittsgruppe wie ein Halogenatom, z.B. ein Chlor-, Brom-oder Jodatom, darstellen, oder dessen Anhydrid in Gegenwart einer Lewis-Säure und gegebenenfalls anschließende Abspaltung eines verwendeten Schutzrestes.

Die Friedel-Craft's-Acylierung wird vorzugsweise in einem Lösungsmittel wie Äthylenchlorid oder Nitrobenzol in Gegenwart einer Lewis-Säure wie Aluminiumchlorid, Bortrifluorid oder Zinkchlorid zweckmäßigerweise bei Temperaturen zwischen 0 und 50°C, vorzugsweise jedoch bei Raumtemperatur, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser Tetrahydrofuran/Wasser oder Dioxan/Wasser, in Gegenwart einer Säure wie Salzsäure oder Schwefelsäure oder in Gegen wart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

d.) Zur Herstellung von Verbindungen der allgemeinen Formel I, in der E benachbart zum Rest B eine Hydroxymethylengruppe enthält:

Reduktion einer Verbindung der allgemeinen Formel

$$R_1-SO_2\overset{\displaystyle R_2}{\overset{|}{N}}-CH_2-A-B-E_3 \qquad ,(VII)$$

in der

$R_1$, $R_2$, A und B wie eingangs definiert sind und $E_3$ die für E eingangs erwähnten Bedeutungen aufweist, wobei jedoch E eine Carbonylgruppe enthalten muß.

Die Reduktion wird in einem geeigneten Lösungsmittel wie Methanol, Äthanol, Äther, Tetrahydrofuran, Dioxan oder Eisessig in Gegenwart von katalytisch angeregtem Wasserstoff, z.B. von Wasserstoff in Gegenwart von Platin oder Palladium/Kohle, und gegebenenfalls in Gegenwart einer

Säure wie Salzsäure oder Perchlorsäure oder in Gegenwart eines Metallhydrids wie Natrium-borhydrid, Lithium-borhydrid oder Lithium-aluminiumhydrid bei Temperaturen zwischen 0 und 100°C, vorzugsweise bei Temperaturen zwischen 20 und 80°C, durchgeführt.

Zur Herstellung einer Verbindung der allgemeinen Formel I, in der E eine Hydroxymethylengruppe enthält, wird die Umsetzung vorzugsweise mit Natrium-borhydrid in Methanol und bei Raumtemperatur durchgeführt.

Enthält eine Verbindung der allgemeinen Formel VII in Rest E eine Doppelbindung, so kann diese bei der Umsetzung mit Wasserstoff in Gegenwart von katalytisch angeregtem Wasserstoff gleichzeitig aufhydriert werden.

e.) Zur Herstellung von Verbindungen der allgemeinen Formel I, in der E einen der eingangs erwähnten gesättigten Reste darstellt:

Hydrierung einer Verbindung der allgemeinen Formel

$$R_1-SO_2\underset{}{\overset{R_2}{\overset{|}{N}}}-CH_2-A-B-E_4 \qquad ,(VIII)$$

in der

$R_1$, $R_2$, A und B wie eingangs definiert sind und $E_4$ einen der für E eingangs erwähnten ungesättigen Rest darstellt.

Die Hydrierung wird in einem geeigneten Lösungsmittel wie Methanol, Äthanol, Dioxan, Essigester oder Eisessig mit katalytisch angeregtem Wasserstoff oder mit nascierendem Wasserstoff bei Temperaturen zwischen 0 und 50°C, vorzugsweise bei Raumtemperatur, durchgeführt. Hierbei kann eine gegebenenfalls im Rest E vorhandene Carbonylgruppe gleichzeitig in eine Hydroxymethylengruppe übergeführt werden.

Zur Herstellung einer Verbindung der allgemeinen Formel I, in der E eine Carbonylgruppe enthält, wird die Umsetzung vorzugsweise in Gegenwart von Zink/Eisessig und bei Raumtemperatur durchgeführt.

f.) Zur Herstellung von Verbindungen der allgemeinen Formel I, in der E eine der eingangs erwähnten über eine gesättigte Alkylengruppe gebundene Hydroxycarbonylgruppe darstellt:

Decarboxylierung einer gegebenenfalls im Reaktionsgemisch hergestellten Verbindung der allgemeinen Formel

$$R_1-SO_2\overset{R_2}{\overset{|}{N}}-CH_2-A-B-E_5 \qquad ,(IX)$$

in der

$R_1$, $R_2$, A und B wie eingangs definiert sind und $E_5$ am gleichen Kohlenstoffatomen zwei über eine geradkettige oder verzweigte Alkylengruppe mit 1 bis 5 Kohlenstoffatomen Kohlenstoffatom gebundene Hydroxycarbonylgruppen darstellt, wobei jeweils eine Methylengruppe des Restes $E_5$, welche mit dem Rest B verknüpft sein muß, durch eine Hydroxymethylen-oder Carbonylgruppe ersetzt ist.

Die Decarboxylierung wird vorzugsweise in Gegenwart einer Säure wie Salzsäure, Schwefelsäure, Bromwasserstoffsäure oder Phosphorsäure, welche gleichzeitig als Lösungsmittel dienen kann, in einem Lösungsmittel wie Wasser, Äthanol/Wasser, Eisessig/Wasser, Dioxan/Wasser oder Diäthylenglykoldimethyläther bei erhöhten Temperaturen, vorzugsweise bei der Siedetemperatur des Reaktionsgemisches, z.B. bei Temperaturen zwischen 80 und 100°C, durchgeführt.

g.) Zur Herstellung von Verbindungen der allgemeinen Formel I, in der E einen Pyridazinonring darstellt:

Umsetzung einer Verbindung der allgemeinen Formel

$$R_1-SO_2\overset{R_2}{\overset{|}{N}}-CH_2-A-B-CO-\overset{W}{\overset{|}{CH}}-\underset{R_7}{\overset{R_6}{C}}-\overset{W}{\overset{|}{CH}}-COOH \qquad ,(X)$$

in der

$R_1$, $R_2$, A und B wie eingangs definiert sind, $R_6$ und $R_7$, die gleich oder verschieden sein können, Wasserstoffatome oder Alkylgruppen, wobei $R_6$ und $R_7$ zusammen insgesamt 1 bis 3 Kohlenstoffatome enthalten können, und W jeweils ein Wasserstoffatom oder zusammen einer weitere Bindung darstellen, oder deren reaktionsfähige Derivate wie deren Ester, Amide oder Halogenide mit Hydrazin.

Die Umsetzung wird zweckmäßigerweise in einem Lösungsmittel wie Methanol, Äthanol, Isopropanol, Eisessig, Propionsäure und/oder in einem Überschuß von Hydrazin bzw. Hydrazin-hydrat bei Temperaturen zwischen 0 und 200°C, z.B. bei Temperaturen zwischen 20 und 150°C, vorzugsweise jedoch bei der Siedetemperatur des Reaktionsgemisches, und gegebenenfalls in Gegenwart einer Säure als Kondensationsmittel wie Schwefelsäure oder p-Toluolsulfonsäure durchgeführt. Die Umsetzung kann jedoch auch ohne Lösungsmittel durchgeführt werden.

h) Zur Herstellung von Verbindungen der allgemeinen Formel I, in der A eine Methylenoxygruppe darstellt:

Umsetzung einer Verbindung der allgemeinen Formel

$$\begin{array}{l} \quad\quad\quad\quad R_2 \\ \quad\quad\quad\quad | \\ R_1-SO_2-N-CH_2-CH_2-Z \end{array} \quad ,(XI)$$

in der

$R_1$ und $R_2$ wie eingangs definiert sind und Z eine nucleophile Austrittsgruppe wie ein Halogenatom oder eine Sulfonyloxygruppe, z.B. ein Chlor-oder Brom, eine Methansulfonyloxy-oder p-Toluolsulfonyloxygruppe, bedeutet, mit einer Verbindung der allgemeinen Formel

HO -B -E ,(XII)

in der

B und E wie eingangs definiert sind, wobei eine im Rest E gegebenenfalls vorhandene Hydroxygruppe durch eine hydrolytisch abspaltbare Schutzgruppe wie eine Alkoxy-oder Benzyloxygruppe geschützt sein kann, gegebenenfalls anschließende Abspaltung eines verwendeten Schutzrestes und gegebenenfalls anschließende Überführung eines erhaltenen Esters der allgemeinen Formel I in die entsprechende Carbonsäure.

Die Umsetzung wird vorzugsweise in einem Lösungsmittel wie Methanol, Äthanol, Wasser/Methanol, Dioxan, Tetrahydrofuran oder Dimethylsulfoxid vorzugsweise in Gegenwart eines wasserfreien säurebindenden Mittels wie Kaliumcarbonat oder Triäthylamin, wobei Triäthylamin auch als Lösungsmittel verwendet werden kann, zweckmäßigerweise bei Temperaturen zwischen 25 und 100°C, vorzugsweise jedoch bei Temperaturen zwischen 40 und 80°C, durchgeführt.

Die gegebenenfalls anschließende Abspaltung eines verwendeten Schutzrestes oder die Überführung eines erhaltenen Esters der allgemeinen Formel I in die entsprechende Carbonsäure erfolgt vorzugsweise hydrolytisch in einem wässrigen Lösungsmittel, z.B. in Wasser, Isopropanol/Wasser Tetrahydrofuran/Wasser oder Dioxan/Wasser, in Gegenwart einer Säure wie Salzsäure oder Schwefelsäure oder in Gegenwart einer Alkalibase wie Natriumhyroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katsalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

i.) Zur Herstellung von Verbindungen der allgemeinen Formel I, in der E eine gegebenenfalls in $\alpha$-Stellung durch eine Methyl-oder Äthylgruppe substituierte Alkoxycarbonylmethylencarbonylgruppe darstellt:

Umsetzung eines Acetophenons der allgemeinen Formel

$$\begin{array}{l} \quad\quad\quad R_2 \\ \quad\quad\quad | \\ R_1-SO_2N-CH_2-A-B-CO-CH_2-R_8 \end{array} \quad ,(XIII)$$

in der

$R_1$, $R_2$, A und B wie eingangs definiert sind und $R_8$ ein Wasserstoffatom, eine Methyl-oder Äthylgruppe darstellt, mit einem Kohlensäuredialkylester in Gegenwart einer Base.

Die Umsetzung wird in Gegenwart einer Base wie Kaliumtert.butylat oder Natriumhydrid und in einem Lösungsmittel wie Toluol, vorzugsweise jedoch in einem Überschuß des eingesetzten Kohlensäuredialkylesters, bei Temperaturen zwischen 50 und 150°C, vorzugsweise bei Temperaturen

zwischen 75 und 100°C, durchgeführt.

Die so erhaltenen neuen Verbindungen der allgemeinen Formel I, falls diese eine Carboxygruppe enthalten, lassen sich gewünschtenfalls anschließend in ihre Additionssalze mit anorganischen oder organischen Basen, insbesondere für die phar mazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangstoffe verwendeten Verbindungen der allgemeinen Formeln II bis XIII erhält man nach literaturbekannten Verfahren bzw. sind literaturbekannt.

Eine als Ausgangsstoff verwendete Verbindung der allgemeinen Formel II erhält man aus einer entsprechenden N-Acyl-amino-alkyl-phenylverbindung durch Acylierung nach Friedel-Craft, anschließende Entacylierung und gegebenenfalls anschließende Reduktion, Hydrolyse und/oder Veresterung.

Die als Ausgangsstoffe verwendeten Verbindungen der allgemeinen Formel IV, V, VII, VIII, IX, X und XIII erhält man durch Umsetzung einer entsprechenden Aminoverbindung mit einem entsprechenden Sulfonylhalogenid.

Wie bereits eingangs erwähnt, weisen die neuen Verbindungen und deren physiologisch verträgliche Additionssalze mit anorganischen oder organischen Basen wertvolle pharmakologische Eigenschaften auf, insbesondere antithrombotische Wirkungen und eine Hemmwirkung auf die Plättchenaggregation. Außerdem weisen sie auch eine Hemmwirkung auf die Tumormetastasierung auf und stellen Thromboxanantagonisten dar. Außerdem weisen die Pyridazinone der allgemeinen Formel I eine Hemmwirkung auf die Phosphodiesterase auf.

Beispielsweise wurden die neuen Verbindungen

A = 3-[4-(2-(4-Chlorbenzolsulfonyl)-aminoäthyl)-benzoyl]-propionsäure,

B = 3-[4-(2-Benzolsulfonylaminoäthyl)-benzoyl]-propionsäure-β-methoxyäthylester,

C = 6-[4-(2-Benzoylsulfonylaminoäthyl)-phenyl]-4,5-dihydro-pyridazin-(2H)-3-on,

D = 6-[4-(2-(p-Toluolsulfonyl)-aminoäthyl)-phenyl]-5-methyl-4,5-dihydro-pyridazin-(2H)-3-on,

E = 6-[4-(2-Benzolsulfonylaminoäthoxy)-phenyl]-4,5-dihydro-pyridazin-(2H)-3-on und

F = 3-[4-(2-Benzolsulfonylamino-äthyl)-benzoyl]-propionsäure auf ihre biologischen Eigenschaften wie folgt geprüft:

1. Antithrombotische Wirkung

Methodik

Die Thrombozytenaggregation wird nach der Methode von BORN und GROSS (J. Physiol. 170, 397 (1964)) in plättchenreichem Plasma gesunder Versuchspersonen gemessen. Zur Gerinnungshemmung wird das Blut mit Natriumchlorid 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ berechnet, die sich auf eine 50%ige Änderung der "optical density" im Sinne einer Aggregationshemmung bezieht.

Die nachfolgende Tabelle enthält die gefundenen Ergebnisse:

| Substanz | $EC_{50}$ [µMol/l] |
|---|---|
| A | 4 |
| B | > 10 |
| C | 3,3 |
| D | 2,4 |
| E | 3 |
| F | 3 |

2. Bestimmung der Verlängerung der Blutungszeit

Vorbemerkung

Der menschliche Organismus sowie der der Warmblütler besitzt einen sinnvollen Mechanismus, der ihn vor Blutverlusten im Falle von Verletzungen schützen soll. Dieses System besteht aus den Blutplättchen (Thrombozyten), welche mittels ihrer Klebeeigenschaften einen Gefäßdefekt rasch "verstopfen" sollen und so die primäre Hämostase herbeiführen. Neben diesem reinen cellulären Blutstillungsmechanismus besitzt der Körper ein Blutgerinnungssystem. Bei diesem System werden Plasmafaktoren (Eiweißkörper) in eine wirksame Form gebracht, welche schließlich das flüssige Plasmafibrinogen zu einem Fibringerinnsel werden lassen.

Das System der primären Hämostase, welches im wesentlichen von den Thrombozyten gestellt wird, und das Gerinnungssystem ergänzen sich in dem gemeinsamen Ziel, den Körper vor Blutverlusten wirkungsvoll zu schützen.

Bei manchen Krankheiten kann es auch bei einem intakten Gefäßsystem zum Ablaufen von Gerinnungsprozessen sowie zum Verklumpen von Thrombozyten kommen. Die Schwächung des Blutgerinnungssystems durch Cumarine oder Heparin ist bekannt und kann leicht mit Hilfe von bekannten Blutgerinnungstesten gemessen werden, welche unter Präparateeinwirkung eine Verlängerung anzeigen - (Plasmarecalcif.-zeit, Quick-Bestimmung, Thrombinzeit, etc.).

Da im Falle einer Verletzung die erste rasche Blutstillung durch Thrombozyten geschieht, läßt sich beim Setzen einer standardisierten Verletzung die Funktion der Thrombozyten mit Hilfe der Messung der Blutungszeit gut bestimmen. Die normale Blutungszeit beträgt beim Menschen etwa 1 bis 3 min., setzt aber leistungsfähige und in genügender Zahl vorhandene Thrombozyten voraus. Bei einer normalen Thrombozytenzahl weist also eine verlängerte Blutungszeit auf eine gestörte Funktion der Thrombozyten hin. Wir finden dies z.B. bei einigen angeborenen Thrombozytenfunktionsstörungen. Will man auf der anderen Seite die Neigung zu spontanem Zusammenballen der Thrombozyten mit der Folge von Gefäßverschlüssen im arteriellen System durch Medikamente verhindern, so muß folglich bei einer erfolgreichen thrombozytenwirksamen Therapie die Blutungszeit unter Substanzeinfluß verlängert werden. Bei einer thrombozytenwirksamen Substanz ist also eine Verlängerung der Blutungszeit und - da das plasmatische Gerinnungssystem ja nicht berührt wird -eine normale Blutgerinnungszeit zu erwarten.

Literatur: W. D. Keidel: Kurzgefaßtes Lehrbuch der Physiologie, Gerog Thieme Verlag Stuttgart 1967, Seite 31: Der Blutstillungsvorgang.

Zur Bestimmung der Blutungszeit wurden die zu untersuchenden Substanzen wachen Mäusen in einer Dosis von 10 mg/kg p.o. appliziert. Nach einer Stunde wurde von der Schwanzspitze jedes Tieres ca. 0,5 mm abgeschnitten und das austretende Blut in Abständen von 30 Sekunden bis zum Sistieren der Blutung vorsichtig mit einem Filterpapier abgetupft. Die Zahl der so erhaltenen Bluttropfen ergibt ein Maß für die Blutungszeit (5 Tiere pro Versuch). Die folgenden Zahlenangaben bedeuten Prozent-Verlängerung gegenüber Kontrollen ohne Substanzgabe:

| Substanz | Verlängerung der Blutungszeit in % nach 1 Stunde |
|---|---|
| A | 108 |
| B | 110 |
| C | 145 |
| D | 187 |
| E | 233 |
| F | >266 |

3. Akute Toxizität

Die akute Toxizität der zu untersuchenden Substanzen wurde orientierend an Gruppen von je 10 Mäusen nach oraler Gabe einer Einzeldosis bestimmt (Beobachtungszeit: 14 Tage):

| Substanz | Orientierende akute Toxizität |
|---|---|
| A | 1.000 mg (0 von 10 Tieren gestorben) |
| B | 1.000 mg (0 von 10 Tieren gestorben) |
| C | 1.000 mg (0 von 10 Tieren gestorben) |
| D | 1.000 mg (0 von 10 Tieren gestorben) |
| E | 1.000 mg (0 von 10 Tieren gestorben) |
| F | 250 mg (0 von 10 Tieren gestorben) |

Aufgrund ihrer pharmakologischen Eigenschaften eignen sich die neuen Verbindungen und deren physiologisch verträgliche Additionssalze zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arterioskerose und zur Metastasenprophylaxe.

Die zur Erzielung einer entsprechenden Wirkung erforderliche Dosierung beträgt zweckmäßigerweise zwei-bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der allgemeinen Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Was ser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Beispiel A

3-[4-(2-Aminoäthyl)-benzoyl]propionsäure-methylester-hydrochlorid

51,5 g (0,2 Mol) 3-[4-(2-Aminoäthyl)-benzoyl]-propionsäure-hydrochlorid werden in 200 ml Methanol suspendiert. Hierzu leitet man Chlorwasserstoff-Gas bis zur Sättigung ein und erhitzt unter Rückfluß 30 Minuten lang. Aus der filtrierten Lösung kristallisieren 31,5 g Esterhydrochlorid aus. Durch Aufarbeitung der Mutterlagen lassen sich weitere 4,4 g erhalten.

Ausbeute: 35,5 g (66 % der Theorie),

Schmelzpunkt: 191 °C

$C_{13}H_{17}NO_3$ x HCl (271,75)

| | | | | | | | | |
|---|---|---|---|---|---|---|---|---|
| Ber.: | C | 57,46 | H | 6,68 | N | 5,15 | Cl | 13,05 |
| Gef.: | | 57,33 | | 6,42 | | 5,02 | | 13,06 |

Beispiel B

3-[4-(2-Aminoäthyl)-benzoyl]buttersäure-äthylester

a.) 4-(2-Acetylaminoäthyl)-2'-chlor-propiophenon

Hergestellt aus N-Acetyl-β-phenäthylamin und 2-Chlorpropionsäurechlorid in Gegenwart von wasserfreiem Aluminiumchlorid.

Ausbeute: 82 % der Theorie,

Schmelzpunkt: 81-85°C.

b.) 3-[4-(Aminoäthyl)-benzoyl]buttersäure

Das gemäß Beispiel Ba) erhaltene Chlorketon wird mit Malonsäurediäthylester und Kalium-tert.butanolat in Dimethylsulf oxid zu 2-Carbäthoxy-3-[4-(2-acetyl-aminoäthyl)-benzoyl]-buttersäure-äthylester umgesetzt. Dieser wird alkalisch hydrolysiert und die daraus gewonnene freie Säure wird durch Erhitzen in Diäthylenglykoldiäthyläther zu 3-[4-(2-Acetyl-amino)-benzoyl]buttersäure vom Schmelzpunkt 147-152°C übergeführt. Anschließend wird durch 6-stündiges Kochen mit halbkonzentrierter Salzsäure 3-[4-(2-Aminoäthyl)-benzoyl]-buttersäure-hydrochlorid erhalten.

c.) Durch Veresterung der gemäß Beispiel Bb) erhaltenen Verbindung mittels alkoholischer Salzsäure erhält man 3-[4-(2-Aminoäthyl)-benzoyl]buttersäure-äthylester.

Öl, Rf-Wert: 0,7 (auf Alox-Platten mit Chloroform-Methanolkonz. Ammoniak (9:1:0,1).

Beispiel C

4-[4-(2-Benzolsulfonylaminoäthyl)-phenyl]-4-hydroxy-buten-(3)-säurelacton

10,8 g 3-[4-(2-Benzolsulfonylaminoäthyl)-benzoyl]-propionsäure werden in 25 ml Acetanhydrid eine Stunde lang zum Sieden erhitzt, wobei nach 10 Minuten eine klare Lösung entsteht. Anschließend destilliert man das Acetanhydrid im Vakuum ab, verreibt den kristallinen Rückstand mit Äther und kristallisiert aus 60 ml Äthylenchlorid um.

Ausbeute: 7,8 g (76 % der Theorie),

Schmelzpunkt: 155-157°C

$C_{18}H_{17}NO_4S$ (343,41)

| | | | | | | | | |
|---|---|---|---|---|---|---|---|---|
| Ber.: | C | 62,96 | H | 4,99 | N | 4,08 | S | 9,34 |
| Gef.: | | 62,85 | | 5,14 | | 4,16 | | 9,18 |

Beispiel D

4-[2-Benzolsulfonylaminoäthyl)-2'-chlor-propiophenon

Hergestellt analog Beispiel B aus N-Benzolsulfonyl-β-phenäthylamin und 2-Chlorpropionsäurechlorid in Gegenwart von wasserfreiem Aluminiumchlorid in Äthylenchlorid.

Beispiel E

4-(2-Benzolsulfonylaminoäthyl)-acetophenon

30 g (0,15 Mol) 4-(2-Aminoäthyl)-acetophenon-hydrochlorid werden in 150 ml Pyridin suspendiert und unter Rühren langsam mit 39,7 g (0,225 Mol) Benzolsulfonsäurechlorid versetzt. Nach einstündigem Rühren versetzt man mit Eis und Salzsäure und saugt das ausgefallene Reaktionsprodukt ab. Es wird aus 200 ml Äthanol und 100 ml Wasser umkristallisiert.

Ausbeute: 20,6 g (44 % der Theorie),

Schmelzpunkt: 138-140°C.

Beispiel F

Benzolsulfonsäure-(N-β-phenyläthyl)-amid

Man unterschichtet eine Lösung von 24,2 g (0,2 Mol) β-Phenäthylamin in 100 ml Dioxan mit 100 ml einer wässrigen Lösung von Kaliumcarbonat (aus 50 ml gesättigtem Kaliumcarbonat und 100 ml Wasser) und tropft unter kräftigem Rühren 42,4 g (0,24 Mol) Benzolsulfochlorid zu. Man rührt 1 Stunde bei Raumtemperatur, saugt den anorganischen Niederschlag ab, trennt die wässrige Phase ab und dampft die Dioxanlösung im Vakuum ein. Den in Chloroform gelösten Rückstand wäscht man mit Wasser. Nach dem Trocknen der organischen Phase wird eingeengt und der erhaltene Rückstand aus 120 ml Toluol und 160 ml Cyclohexan umkristallisiert.

Ausbeute: 41,0 g (78 % der Theorie),

Schmelzpunkt: 66-68°C

Beispiel G

3-[4-(2-Acetylaminoäthyl)-benzoyl]propionsäure

Man suspendiert 504,3 g (3,78 Mol) wasserfreies Aluminiumchlorid in 1,3 l Äthylenchlorid und versetzt unter Rühren und Kühlen mit Eiswasser nacheinander mit 189,2 g (1,89 Mol) Bernsteinsäureanhydrid und 280,2 g (0,172 Mol) N-Acetyl-β-phenyläthylamin. Man rührt 2 Stunden bei Zimmertemperatur und versetzt anschließend mit Eis und 550 ml konzentrierter Salzsäure. Es fällt ein Niederschlag aus, welcher abgesaugt und zwischen Chloroform und 2n-Natriumhydroxid verteilt wird. Die alkalisch-wässrige Phase wird angesäuert. Die ausfallende Säure wird abgesaugt und aus 1200 ml Wasser umkristallisiert.

Ausbeute: 109 g (24 % der Theorie),

Schmelzpunkt: 145-147°C.

Beispiel H

3-[4-(2-Aminoäthyl)-benzoyl]propionsäure-hydrochlorid

55,9 g 3-[4-(2-Acetylaminoäthyl)-benzoyl]propionsäure werden in 200 ml halbkonzentrierter Salzsäure 22 Stunden im Ölbad gekocht. Man dampft die klare Lösung im Vakuum eine und kristallisiert den Rückstand aus 200 ml n-Propanol um.

Ausbeute: 49,8 g (92 % der Theorie),

Schmelzpunkt: 163-166°C. (Zers.)

Beispiel 1

3-[4-(2-Benzolsulfonylaminoäthyl)-benzoyl]propionsäure-Kaliumsalz

Zu einer Lösung von 10,8 g (0,03 Mol) 3-[4-(2-Benzolsulfonylaminoäthyl)-benzoyl]propionsäure in 100 ml Methanol gibt man eine Lösung von 1,9 g (0,033 Mol) Kaliumhydroxid in 25 ml Methanol und versetzt anschließend mit 400 ml Äther. Das kristallin ausfallende Kaliumsalz wird nach einer Stunde abgesaugt und nach dem Trocknen aus 200 ml n-Propanol umkristallisiert.

Ausbeute: 9,3 g (78 % der Theorie),

Schmelzpunkt: 165-167°C

$C_{18}H_{18}KNO_5$ (399,52)

| | | | | |
|---|---|---|---|---|
| Ber.: | N | 3,51 | S | 8,03 |
| Gef.: | | 3,39 | | 8,05 |

Beispiel 2

3-[4-(2-(4-Fluorbenzolsulfonyl)-aminoäthyl)-benzoyl]-propionsäure-methylester

13,6 g (0,05 Mol) 3-[4-(2-Aminoäthyl)-benzoyl-propionsäure-methylester-hydrochlorid werden in 70 ml Pyridin suspendiert. Man gibt 10,6 g (0,1 Mol) Triäthylamin und 200 mg 4-Dimethylaminopyridin und dann, unter Kühlung mit Eis-Kochsalz, 11,7 g (0,06 Mol) 4-Fluorbenzolsulfonsäurechlorid zu. Man rührt anschließend eine Stunde bei Raumtemperatur. Zur Aufarbeitung verdünnt man mit Eiswasser, versetzt mit Salzsäure und saugt das Reaktionsprodukt ab. Man kristallisiert aus 50 ml Methanol um.

Ausbeute: 18,7 g (94 % der Theorie),

Schmelzpunkt: 92-94°C

$C_{19}H_{20}FNO_5S$ (393,30)

| | | | | |
|---|---|---|---|---|
| Ber.: | N | 3,56 | S | 8,15 |
| Gef.: | | 3,63 | | 8,24 |

Beispiel 3

3-[4-(2-(4-Fluorbenzolsulfonyl)-aminoäthyl)-benzoyl]-propionsäure

9,8 g (0,025 Mol) des gemäß Beispiel 2 erhaltenen Ester werden mit 30 ml wässriger 2 n Natriumhydroxidlösung zum Sieden erhitzt. Dabei entsteht eine klare Lösung. Man säuert an und saugt das Reaktionsprodukt ab. Nach dem Trocknen kristallisiert man aus 60 ml n-Propanol um.

Ausbeute: 8,0 g (84 % der Theorie),

Schmelzpunkt: 132-134°C

$C_{18}H_{18}FNO_5S$ (379,42)

| | | | | | | | | |
|---|---|---|---|---|---|---|---|---|
| Ber.: | C | 56,98 | H | 4,78 | N | 3,69 | S | 8,45 |
| Gef.: | | 56,74 | | 4,77 | | 3,82 | | 8,41 |

Beispiel 4

3-[4-(2-(4-Chlorbenzolsulfonyl)-aminoäthyl)-benzoyl]-propionsäure-methylester

Hergestellt analog Beispiel 2 aus 3-[4-(2-Aminoäthyl)-benzoyl]propionsäure-methylester-hydrochlorid und 4-Chlorbenzolsulfonsäurechlorid.

Schmelzpunkt: 111-113°C (aus Methanol/Wasser)

$C_{19}H_{20}ClNO_5S$ (409,90)

| | | | | | | | | |
|---|---|---|---|---|---|---|---|---|
| Ber.: | C | 55,67 | H | 4,92 | N | 3,42 | S | 7,82 |
| Gef.: | | 55,67 | | 4,98 | | 3,24 | | 7,59 |

Beispiel 5

3-[4-(2-(4-Chlorbenzolsulfonyl)-aminoäthyl)-benzoyl]-propionsäure

Hergestellt analog Beispiel 3 aus 3-[4-(2-(4-Chlorbenzolsulfonyl)-aminoäthyl)-benzoyl]-propionsäure-methylester.

Schmelzpunkt: 150-152°C (aus Tetrachlorkohlenstoff)

$C_{18}H_{18}ClNO_5S$ (395,87)

| | | | | | | | | | | |
|---|---|---|---|---|---|---|---|---|---|---|
| Ber.: | C | 54,61 | H | 4,58 | Cl | 8,96 | N | 3,54 | S | 8,10 |
| Gef.: | | 54,90 | | 4,66 | | 9,26 | | 3,30 | | 8,22 |

Beispiel 6

3-[4-(2-(p-Toluolsulfonyl)-aminoäthyl)-benzoyl]propionsäure-methylester

Hergestellt analog Beispiel 2 aus 3-[4-(2-Aminoäthyl)-benzoyl]propionsäuremethylester-hydrochlorid und Toluolsulfonsäurechlorid.

Schmelzpunkt: 120-122°C (aus Methanol/Wasser)

Beispiel 7

3-[4-(2-(p-Toluolsulfonyl)-aminoäthyl)-benzoyl]propionsäure

Hergestellt analog Beispiel 3 aus 3-[4-(2-(p-Toluolsulfonyl)-aminoäthyl)-benzoyl]propionsäure-methylester.

Schmelzpunkt: 130-132°C

$C_{19}H_{21}NO_5S$ (375,45)

| | | | | | | | | |
|---|---|---|---|---|---|---|---|---|
| Ber.: | C | 60,78 | H | 5,64 | N | 3,73 | S | 8,54 |
| Gef.: | | 60,42 | | 5,62 | | 3,91 | | 8,61 |

Beispiel 8

3-[4-(2-(3-Pyridinsulfonyl)-aminoäthyl)-benzoyl]-propionsäure-methylester

Hergestellt analog Beispiel 2 aus 3-[4-(2-Aminoäthyl)-benzoyl]propionsäure-methylester-hydrochlorid und 3-Pyridinsulfonsäurechlorid.

Ausbeute: 85 % der Theorie.

Schmelzpunkt: 111-113°C

$C_{18}H_{20}N_2O_5S$ (376,344)

| | | | | | | | | |
|---|---|---|---|---|---|---|---|---|
| Ber.: | C | 57,43 | H | 5,36 | N | 7,44 | S | 8,52 |
| Gef.: | | 57,20 | | 5,33 | | 7,49 | | 8,48 |

Beispiel 9

3-[4-(2-(3-Pyridinsulfonyl)-aminoäthyl)-benzoyl]propionsäure

Hergestellt analog Beispiel 3 aus 3-[4-(2-(3-Pyridinsulfonyl)-aminoäthyl)-benzoyl]propionsäure-methylester.

Ausbeute: 71 % der Theorie.

Schmelzpunkt; 158-160°C (aus n-Propanol)

$C_{17}H_{18}N_2O_5S$ (362,42)

| | | | | | | | | |
|---|---|---|---|---|---|---|---|---|
| Ber.: | C | 56,34 | H | 5,01 | N | 7,73 | S | 8,85 |
| Gef.: | | 56,09 | | 5,02 | | 7,53 | | 8,83 |

Beispiel 10

3-[4-(2-(2-Thiophensulfonyl)-aminoäthyl)-benzoyl]-propionsäure-methylester

Hergestellt analog Beispiel 2 aus 3-[4-(2-Aminoäthyl)-benzoyl]propionsäure-methylester-hydrochlorid und 2-Thiophen-sulfonsäurechlorid.

Ausbeute: 80 % der Theorie,

Öl, Rf-Wert: 0,2 (Kieselgel-Polygram-Platten mit Cyclohexan-Essigester = 2:1).

Beispiel 11

3-[4-(2-(2-Thiophensulfonyl)-aminoäthyl)-benzoyl]-propionsäure

Hergestellt analog Beispiel 3 aus 3-[4-(2-(2-Thiophensulfonyl)-aminoäthyl)-benzoyl]-propionsäure-methylester.

Ausbeute: 60 % der Theorie,

Schmelzpunkt: 141-143°C (aus Isopropanol)

$C_{16}H_{17}NO_5S_2$ (367,46)

| | | | | | | |
|---|---|---|---|---|---|---|
| Ber.: | C | 52,30 | H | 4,66 | N | 3,81 |
| Gef.: | | 52,31 | | 4,89 | | 3,94 |

Beispiel 12

3-4-(2-(2'-Fluor-4-biphenylyl-sulfonyl)-aminoäthyl)-benzoyl]-propionsäure

Eine Suspension von 7,7 g (0,03 Mol) 3-[4-(2-Aminoäthyl)-benozyl]propionsäure-hydrochlorid in 25 ml Pyridin versetzt man mit 100 mg 4-Dimethylaminopyridin und dann unter Eiskühlung mit 9,7 g (0,036 Mol) 2'-Fluor-4-biphenylsulfonsäurechlorid. Nach einer Stunde verdünnt man mit Wasser, säuert an und kristallisiert das Reaktionsprodukt aus Äthylenchlorid um.

Ausbeute: 7,7 g (56 % der Theorie),

Schmelzpunkt: 160-162°C

$C_{24}H_{22}FNO_5S$ (455,51)

| | | | | | | | | |
|---|---|---|---|---|---|---|---|---|
| Ber.: | C | 63,28 | H | 4,87 | N | 3,08 | S | 7,07 |
| Gef.: | | 63,44 | | 4,95 | | 3,28 | | 7,36 |

Beispiel 13

4-[4-(2-Benzolsulfonylaminoäthyl)-phenyl]-4-hydroxy-buttersäure

7,2 g (0,02 Mol) 3-[4-(2-Benzolsulfonylaminoäthyl)-benzoyl]-propionsäure werden in 15 ml 2n Natriumhydroxid gelöst und mit 0,4 g (0,01 Mol) Natriumborhydrid versetzt. Man rührt eine Stunde bei Raumtemperatur, säuert anschließend an und extrahiert das Reaktionsprodukt mit Essigester. Dieses wird durch Chromatographie an einer Lobar[(R)]-Fertigsäule der Firma Merck A.G. mit Äthylenchlorid-Äthanol (25:1) chromatographiert.

Ausbeute: 4,0 g (55 % der Theorie),

Öl, Rf-Wert: 0,3 (Kieselgel-Polygram-Platten mit Äthylenchlorid-Äthanol 10:1).

| | | | | | | | | |
|---|---|---|---|---|---|---|---|---|
| Ber.: | C | 59,49 | H | 5,83 | N | 3,85 | S | 8,82 |
| Gef.: | | 59,40 | | 5,59 | | 3,68 | | 8,67 |

Beispiel 14

3-[4-(2-Benzolsulfonylaminoäthyl)-benzoyl]acrylsäure

Man suspendiert 60 g (0,45 Mol) wasserfreies Aluminiumchlorid in 120 ml Äthylenchlorid und gibt unter kräftigem Rühren und Kühlen mit Eiswaser nacheinander 22,1 g (0,225 Mol) Maleinsäureanhydrid und 39,2 g (0,15 Mol) Benzolsulfonsäure-(N-β-phenyläthyl)-amid zu. Man rührt 2 Stunden bei Raumtemperatur, versetzt mit Eis, destilliert das Lösungsmittel mit Wasserdampf ab und isoliert das Reaktionsprodukt.

Ausbeute: 87 % der Theorie,

Schmelzpunkt: 139-141 °C (aus Äthylenchlorid)

$C_{18}H_{17}NO_5S$ (359,41)

| | | | | | | | | |
|---|---|---|---|---|---|---|---|---|
| Ber.: | C | 60,15 | H | 4,77 | N | 3,90 | S | 8,92 |
| Gef.: | | 60,20 | | 4,72 | | 4,07 | | 8,89 |

Beispiel 15

3-[4-(2-Benzolsulfonylaminoäthyl)-benzoyl]buttersäureäthylester

Hergestellt analog Beispiel 2 aus 3-[4-(2-Aminoäthyl)-benzoyl]buttersäureäthylester und Benzolsulfonsäurechlorid in Pyridin.

Öl, Rf-Wert: 0,6 (Kieselgel-Polygram-Platten mit CyclohexanEssigester 1:1).

Beispiel 16

3-[4-(2-Benzolsulfonylaminoäthyl)-benzoyl]buttersäure

Hergestellt analog Beispiel 3 aus 3-[4-(2-Benzolsulfonyl-aminoäthyl)-benzoyl]buttersäure-äthylester.

Ausbeute: 90 % der Theorie,

Schmelzpunkt: 84-87 °C (aus Diisopropyläther)

$C_{19}H_{21}NO_5S$ (375,45)

| | | | | | | | | |
|---|---|---|---|---|---|---|---|---|
| Ber.: | C | 60,78 | H | 5,64 | N | 3,73 | S | 8,54 |
| Gef.: | | 61,00 | | 5,51 | | 3,66 | | 8,41 |

Beispiel 17

3-[4-(2-p-Chlorbenzolsulfonylaminoäthyl)-benzoyl]-buttersäure

Hergestellt analog Beispiel 2 aus 3-[4-(2-Aminoäthyl)-benzoyl]buttersäure-äthylester und 4-Chlorbenzolsulfonsäurechlorid in Pyridin und Triäthylamin und anschließender Hydrolyse.

Ausbeute: 56 % der Theorie,

Schmelzpunkt: 65-68 °C (aus Dioxan-Cyclohexan).

Beispiel 18

3-[4-2-p-Toluolsulfonylaminoäthyl)-benzoyl]buttersäureäthylester

Hergestellt analog Beispiel 2 aus 3-[4-(2-Aminoäthyl)-benzoyl]buttersäure-äthylester.

Öl, Rf-Wert: 0,6 (Merck-Kieselgel-Platten mit CyclohexanEssigester 1:1 als Laufmittel).

Beispiel 19

3-[4-(2-p-Toluolsulfonylaminoäthyl)-benzoyl]buttersäure

Hergestellt analog Beispiel 3 aus 3-[4-(2-p-Toluolsulfonyl-aminoäthyl)-benzoyl]buttersäure-äthylester.

Ausbeute: 55 % der Theorie,

Schmelzpunkt: 109-112°C (aus Essigester-Diisopropyläther).

$C_{20}H_{23}NO_5S$ (389,47)

| | | | | | | | | |
|---|---|---|---|---|---|---|---|---|
| Ber.: | C | 61,68 | H | 5,95 | N | 3,60 | S | 8,23 |
| Gef.: | | 61,90 | | 6,16 | | 3,63 | | 8,01 |

Beispiel 20

3-[4-(2-(3-Pyridylsulfonyl)-aminoäthyl)-benzoyl]buttersäure

Hergestellt ananlog Beispiel 2 aus 3-[4-(2-Aminoäthyl)-benzoyl]buttersäure-äthylester und Pyridin-3-sulfonsäurechlorid und anschließende Hydrolyse.

Ausbeute: 37 % der Theorie,

Schmelzpunkt: 130-132°C (aus Essigester).

$C_{18}H_{20}N_2O_5S$ (376,43)

| | | | | | | | | |
|---|---|---|---|---|---|---|---|---|
| Ber.: | C | 57.43 | H | 5,36 | N | 7.44 | S | 8.52 |
| Gef.: | | 57,20 | | 5,38 | | 7,20 | | 8,52 |

Beispiel 21

6-[4-2-Benzolsulfonylaminoäthyl)-phenyl]-4,5-dihydro-pyridazin-(2H)-3-on

Zu einer Suspension von 54,2 g (0,15 Mol) 3-[4-(2-Benzolsulfonylaminoäthyl)-benzoyl]propionsäure in 200 ml Eisessig tropft man 41,3 g (0,825 Mol) 99%iges Hydrazinhydrat und erhitzt anschließend für eine Stunde zum Sieden. Danach kühlt man ab. Man isoliert das auskristallisierte Reaktionsprodukt und reinigt es durch Umkristallisation aus n-Butanol.

Ausbeute: 43,6 g (81 % der Theorie),

Schmelzpunkt: 173-175°C

$C_{18}H_{19}N_3O_3S$ (357,44)

| | | | | | | | | |
|---|---|---|---|---|---|---|---|---|
| Ber.: | C | 60,48 | H | 5,36 | N | 11,76 | S | 8,97 |
| Gef.: | | 60,90 | | 5,32 | | 11,80 | | 9,10 |

Beispiel 22

6-[4-(2-(4-Fluorbenzolsulfonyl)-aminoäthyl)-phenyl]-4,5-dihydro-pyridazin-(2H)-3-on

Hergestellt analog Beispiel 21 aus 3-[4-(2-(4-Fluorbenzolsulfonyl)-aminoäthyl)-benzoyl]propionsäure und Hydrazinhydrat in Eisessig.

Ausbeute: 65 % der Theorie,

Schmelzpunkt: 172-174°C (aus n-Butanol)

$C_{18}H_{18}FN_3O_3S$ (375.43)

| | | | | | | | | |
|---|---|---|---|---|---|---|---|---|
| Ber.: | C | 57,59 | H | 4,83 | N | 11,19 | S | 8,54 |
| Gef.: | | 57,90 | | 4,92 | | 11,30 | | 8,68 |

Beispiel 23

6-[4-(2-Benzolsulfonylaminoäthyl)-phenyl]-5-methyl-4,5-dihydro-pyridazin-(2H)-3-on

Hergestellt analog Beispiel 21 aus 3-[4-(2-Benzolsulfonyl-aminoäthyl)-benzoyl]buttersäure und Hydrazinhydrat in Eisessig.

Ausbeute: 75 % der Theorie,

Schmelzpunkt: 120-125°C (aus Isopropanol-Tetrachlorkohlenstoff).

Beispiel 24

6-[4-(2-p-Toluolsulfonyl)-aminoäthyl)-phenyl]-5-methyl-4,5-dihydro-pyridazin-(2H)-3-on

7,0 g 3-[4-(2-p-Toluolsulfonyl)-aminoäthyl)-benzoyl]-buttersäure-äthylester und 2 g 99%iges Hydrazinhydrat werden in 25 ml Eisessig 5 Stunden gekocht. Man dampft im Vakuum ein, verteilt den Rückstand zwischen wässriger Natriumcarbonat-Lösung und Methylenchorid. Die organische Phase wird eingedampft und der Rückstand aus 65 ml Essigester umkristallisiert.

Ausbeute: 4,0 g (62 % der Theorie),

Schmelzpunkt: 147-150°C

$C_{20}H_{23}N_3O_3S$ (385,49).

| | | | | | | | | |
|---|---|---|---|---|---|---|---|---|
| Ber.: | C | 62,32 | H | 6,01 | N | 10.90 | S | 8,32 |
| Gef.: | | 62,48 | | 5,97 | | 10,77 | | 8,32 |

Beispiel 25

6-[4-(2-(p-Chlorbenzolsulfonyl)-aminoäthyl)-phenyl]-5-methyl-4,5-dihydro-pyridazin-(2H)-3-on

Hergestellt analog Beispiel 24 aus 3-[4-(2-p-Chlorbenzolsulfonyl)-aminoäthyl)-benzoyl]-buttersäure-äthylester und Hydrazinhydrat in Eisessig.

Ausbeute: 48 % der Theorie,

Schmelzpunkt: 152-154°C.

Beispiel 26

6-[4-(2-(3-Pyridinsulfonyl)-aminoäthyl)-phenyl]-5-methyl-4,5-dihydro-pyridazin-(2H)-3-on

Hergestellt analog Beispiel 24 aus 3-[4-(2-(3-Pyridinsulfonyl)-aminoäthyl)-benzoyl]buttersäure-äthylester und Hydrazinhydrat in Eisessig.

Ausbeute: 40 % der Theorie,

Schmelzpunkt: 120-122°C (aus Isopropanol)

$C_{18}H_{20}N_4O_3S$ (372,46)

| | | | | | | | | |
|---|---|---|---|---|---|---|---|---|
| Ber.: | C | 58,05 | H | 5,41 | N | 15,04 | S | 8,61 |
| Gef.: | | 58,00 | | 5,41 | | 14,51 | | 8,53 |

Beispiel 27

3-[4-(2-N-Benzolsulfonyl-N-methyl-aminoäthyl)-benzoyl]-propionsäure

Hergestellt analog Beispiel 14 aus N-Benzolsulfonyl-N-methyl-$\beta$-phenyläthylamin und Bernsteinsäureanhydrid in Ge genwart von Aluminiumchlorid in Äthylenchlorid.

Ausbeute: 37 % der Theorie.

Schmelzpunkt: 123-125°C (aus Isopropanol)

$C_{19}H_{21}NO_5S$ (375,45)

| | | | | | | | | |
|---|---|---|---|---|---|---|---|---|
| Ber.: | C | 60,78 | H | 5,64 | N | 3,73 | S | 8,54 |
| Gef.: | | 60,82 | | 5,62 | | 3,61 | | 8,65 |

Beispiel 28

6-[4-(2-N-Benzolsulfonyl-N-methyl-aminoäthyl)-phenyl]-4,5-dihydro-pyridazin-(2H)-3-on

Hergestellt analog Beispiel 21 aus 3-4-(2-N-Benzolsulfonyl-N-methyl-aminoäthyl)-benzoyl]propionsäure und Hydrazinhydrat in Eisessig.

Ausbeute: 66 % der Theorie,

Schmelzpunkt: 181-183°C

$C_{19}H_{21}N_3O_3S$ (371,47)

| | | | | | | | | |
|---|---|---|---|---|---|---|---|---|
| Ber.: | C | 61,43 | H | 5,70 | N | 11,31 | S | 8,63 |
| Gef.: | | 61,40 | | 5,85 | | 11,38 | | 8,70 |

Beispiel 29

3-[4-(2-Benzolsulfonylaminoäthoxy)-benzoyl]propionsäure-methylester

9,8 g (37 mMol) N-Benzolsulfonyl-N-$\beta$-bromäthyl-amin (J. Indian chem. Soc. 17, 567 (1940)) und 7,7 g (37 mMol) 3-[4-Hydroxybenzoyl)-propionsäure-methylester (Schmelzpunkt: 119-120°C; J. Chem. Soc. London 1944, 548) werden zusammen mit 5,1 g trockenem Kaliumcarbonat in 50 ml Dimethylsulfoxid 6 Stunden lang auf 50-60°C unter Rühren erhitzt. Man ver setzt anschließend mit 150 ml Wasser, dekantiert von dem ausgefallenen Öl ab, extrahiert dieses mit Essigester und wäscht die organische Phase mit verdünnter Natriumhydroxid-Lösung. Nach Trocknen und Eindampfen erhält man 3-[4-(2-Benzolsulfonylaminoäthoxy)-benzoyl]propionsäure-methylester als Öl.

Rf-Wert: 0,6 (Kieselgel-Fertigplatten der Firma Merck mit Äthylenchlorid, Essigester, Eisessig = 100:30:5 als Laufmittel).

Ausbeute: 11,8 g (81 % der Theorie).

Beispiel 30

3-[4-(2-Benzolsulfonylaminoäthoxy-benzoyl]propionsäure

Hergestellt analog Beispiel 3 durch alkalische Hydrolyse von 3-[4-(2-Benzolsulfonylaminoäthoxy)-benzoyl]-propionsäure-methylester.

Ausbeute: 9,8 g (86 % der Theorie),

Schmelzpunkt: 139-141°C (aus n-Butanol)

$C_{18}H_{19}NO_6S$ (377,43)

| | | | | | | | | |
|---|---|---|---|---|---|---|---|---|
| Ber.: | C | 57,28 | H | 5,07 | N | 3,71 | S | 8,50 |
| Gef.: | | 57,31 | | 5,16 | | 4,07 | | 8,82 |

Beispiel 31

3-[4-(2-p-Toluolsulfonylaminoäthoxy)-benzoyl]propionsäure

Hergestellt analog Beispiel 29 aus N-$\beta$-Toluolsulfonyl-N-$\beta$-bromäthyl-amin und 3-(4-Hydroxybenzoyl)-propionsäure-methylester und anschließende alkalische Hydrolyse des 3-[4-(2-p-Toluolsulfonylaminoäthoxy)-benzoyl]-propionsäure-methylester.

Ausbeute: 32 % der Theorie,

Schmelzpunkt: 166-167°C (aus n-Butylacetat).

Beispiel 32

6-[4-(2-Benzolsulfonylaminoäthoxy)-phenyl]-4,5-dihydropyridazin-(2H)-3-on

Hergestellt analog Beispiel 21 aus 3-[4-(2-Benzolsulfonylaminoäthoxy)-benzoyl]propionsäure und Hydrazinhydrat in Eisessig.

Ausbeute: 87 % der Theorie,

Schmelzpunkt: 156-157°C (aus n-Butanol).

Beispiel 33

5-[4-(2-Benzolsulfonylaminoäthyl)-benzoyl]pentansäure-äthylester

Zu einer Suspension von 16,7 g (125 mMol) wasserfreiem Aluminiumchlorid in 80 ml Äthylenchlorid gibt man 9,3 g (48 mMol) Adipinsäurechlorid-monoäthylester und anschließend 12,6 g (48 mMol) N-Benzolsulfonyl-N-β-phenäthyl-amin und rührt 4 Stunden bei Raumtemperatur. Man versetzt mit Eis und Salzsäure, isoliert das Reaktionsprodukt und reinigt es durch Säulenchromatographie an 650 g Kieselgel mit Cyclohexan-Essigester 1:1.

Ausbeute: 12,5 g (62 % der Theorie),

Öl, Rf-Wert; 0,6 (Kieselgel-Polygram-Platten mit Cyclohexan-Essigester 1:1).

Beispiel 34

5-[4-(2-Benzolsulfonylaminoäthyl)-benzoyl]pentansäure

5,5 g (13 mMol) 5-[4-(2-Benzolsulfonylaminoäthyl)-benzoyl]pentansäure-äthylester werden mit Natriumhydroxid in Äthanol 2 Stunden gekocht. Man dampft ein, nimmt in Wasser auf, wäscht die wässrige Lösung mit Äther und säuert sie an. Das ausgefallene Reaktionsprodukt wird aus 40 ml Essigester umkristallisiert.

Ausbeute; 2,4 g (47 % der Theorie),

Schmelzpunkt: 129-130°C

$C_{20}H_{23}NO_5S$ (389,47)

| | | | | | | | | |
|---|---|---|---|---|---|---|---|---|
| Ber.: | C | 61,68 | H | 5,95 | N | 3,60 | S | 8,23 |
| Gef.: | | 61,55 | | 5,87 | | 3,50 | | 8,18 |

Beispiel 35

3-[4-(2-(1-Naphthalinsulfonyl)-aminoäthyl)-benzoyl]-propionsäure-methylester

Hergestellt analog Beispiel 2 aus 3-[4-(2-Aminoäthyl)-benzoyl]propionsäure-methylester-hydrochorid und 1-Naphthalinsulfonsäurechlorid in Pyridin und Triäthylamin.

Ausbeute: 66 % der Theorie,

Schmelzpunkt: 130-132°C (aus n-Propanol-Wasser).

Beispiel 36

3-[4-(2-(1-Naphthalinsulfonyl)-aminoäthyl)-benzoyl]-propionsäure

Hergestellt durch alkalische Hydrolyse von 3-[4-(2-(1-Naphthalinsulfonyl)-aminoäthyl)-benzoyl]propionsäure-methylester.

Ausbeute: 56 % der Theorie,

Schmelzpunkt: 156-158°C (aus n-Propanol-Wasser

$C_{22}H_{21}NO_5S$ (411,48)

| | | | | | | | | |
|---|---|---|---|---|---|---|---|---|
| Ber.: | C | 64,22 | H | 5,15 | N | 3,40 | S | 7,79 |
| Gef.: | | 64,30 | | 5,12 | | 3,26 | | 8,07 |

Beispiel 37

3-[4-(2-Benzolsulfonylaminoäthyl)-naphthoyl-(1)-]propionsäure

Hergestellt analog Beispiel 14 aus N-Benzolsulfonyl-N-β-1-naphthyläthyl-amin und Bernsteinsäureanhydrid in Äthylenchlorid in Gegenwart von Aluminiumchlorid.

Ausbeute: 35 % der Theorie,

Schmelzpunkt: 53-56°C (aus Methanol/Wasser).

Beispiel 38

3-[4-(2-Benzolsulfonylaminoäthyl)-benzoyl]propionsäure-äthyl-ester

7,8 g 4-[4-(2-Benzolsulfonylamino-äthyl)-phenyl]-4-hydroxy-buten-(3)-säurelacton werden in 20 ml Äthanol in Gegenwart einer katalytischen Menge p-Toluolsulfonsäure eine Stunde gekocht. Man dampft ein, löst den Rückstand in Chloroform, wäscht mit Natriumhydrogencarbonatlösung und Wasser, trocknet und dampft ein. Man kristallisiert aus 25 ml Toluol um.

Ausbeute: 7,0 g (80 % der Theorie),

Schmelzpunkt: 85-87°C

$C_{20}H_{23}NO_5S$ (389,48)

| | | | | | | | | |
|---|---|---|---|---|---|---|---|---|
| Ber.: | C | 61,68 | H | 5,95 | N | 3,60 | S | 8,23 |
| Gef.: | | 61,94 | | 5,91 | | 3,62 | | 8,08 |

Beispiel 39

3-[4-(2-Benzolsulfonylaminoäthyl)-benzoyl]propionsäure-methylester

Hergestellt analog Beispiel 38 aus 4-[4-(2-Benzolsulfonylaminoäthyl)-phenyl]-4-hydroxy-buten-(3)-säurelacton und Methanol.

Ausbeute: 84 % der Theorie,

Schmelzpunkt: 92-94°C (aus Methanol)

$C_{19}H_{21}NO_5S$ (375,45)

| | | | | |
|---|---|---|---|---|
| Ber.: | N | 3,73 | S | 8,54 |
| Gef.: | | 3,77 | | 8,30 |

Beispiel 40

3-[4-(2-Benzolsulfonylaminoäthyl)-benzoyl]propionsäure-β-methoxyäthylester

Hergestellt analog Beispiel 38 aus 4-[4-(2-Benzolsulfonylaminoäthyl)-phenyl]-4-hydroxy-buten-(3)-säurelacton und Glykol-monomethyläther.

Ausbeute: 63 % der Theorie,

Schmelzpunkt: 62-64°C (aus Essigester/Diisopropyläther)

$C_{21}H_{25}NO_6S$ (419,50)

| | | | | | | | | |
|---|---|---|---|---|---|---|---|---|
| Ber.: | C | 60,13 | H | 6,01 | N | 3,34 | S | 7,64 |
| Gef.: | | 60,17 | | 6,12 | | 3,45 | | 7,59 |

Beispiel 41

6-[4-(2-(4-Chlorbenzolsulfonyl)-aminoäthyl)-phenyl]-4,5-dihydro-pyridazin-(2H)-3-on

Hergestellt analog Beispiel 21 aus 3-[4-(2-(4-Chlorbenzolsulfonyl)-aminoäthyl)-benzoyl]propionsäure und Hydrazinhydrat in Eisessig.

Ausbeute: 68 % der Theorie,

Schmelzpunkt: 174-176°C (aus n-Propanol)

$C_{18}H_{18}ClN_3O_3S$ (391,89)

| | | | | | | | | | | |
|---|---|---|---|---|---|---|---|---|---|---|
| Ber.: | C | 55,17 | H | 4,63 | N | 10,72 | S | 8,18 | Cl | 9,05 |
| Gef.: | | 55,24 | | 4,85 | | 10,62 | | 8,26 | | 9,10 |

Beispiel 42

6-[4-(2-(p-Toluolsulfonyl)-aminoäthyl)-phenyl]-4,5-dihydro-pyridazin-(2H)-3-on

Hergestellt analog Beispiel 21 aus 3-[4-(2-p-Toluolsulfonyl)-aminoäthyl)-benzoyl]propionsäure und Hydrazin in Eisessig.

Ausbeute: 54 % der Theorie,

Schmelzpunkt: 169-171 °C (aus n-Propanol)

$C_{19}H_{21}N_3O_3S$ (371,47)

| | | | | | | | | |
|---|---|---|---|---|---|---|---|---|
| Ber.: | C | 61,44 | H | 5,70 | N | 11,31 | S | 8,63 |
| Gef.: | | 61,42 | | 5,40 | | 11,70 | | 8,49 |

Beispiel 43

6-[4-(2-Benzolsulfonylaminoäthyl)-naphthyl]-(1)]-4,5-dihydro-pyridazin-(2H)-3-on

Hergestellt analog Beispiel 21 aus 3-[4-(2-Benzolsulfonylaminoäthyl)-naphthoyl-(1)]-propionsäure und Hydrazinhydrat in Eisessig.

Ausbeute: 43 % der Theorie,

Öl, RF-Wert: 0,45 (Kieselgel-Polygram-Platten mit Benzol-Äthanol-konz. Ammoniak = 80:20:1).

Beispiel 44

3-[4-(2-Benzolsulfonylaminoäthyl)-benzoyl]propionsäure

Man löst 3,6 g (0,01 Mol) 3-[4-(2-Benzolsulfonylaminoäthyl)-benzoyl]acrylsäure in 100 ml Äthanol, versetzt mit Raney-Nickel und hydriert bei Raumtemperatur unter 5 bar Wasserstoffdruck. Nach Aufnahme von 1 Äquivalent Wasserstoff kommt die Reaktion zum Stillstand. Man saugt vom Katalysator ab, dampft ein und kristallisiert den Rückstand aus 20 ml Äthanol um.

Ausbeute: 2,6 g (72 % der Theorie),

Schmelzpunkt: 152-154 °C.

Beispiel 45

3-[4-(2-Benzolsulfonylaminoäthyl)-benzoyl]propionsäure

3,6 g (0,01 Mol) 3-[4-(2-Benzolsulfonylaminoäthyl)-benzoyl]-acrylsäure werden in 50 ml Eisessig mit 3 g Zinkstaub ver setzt und auf dem Dampfbad unter gelegentlichem Schütteln 5 Stunden lang erhitzt. Man filtriert vom nicht umgesetzten Zink ab, versetzt das Filtrat mit 100 ml Wasser, saugt das Reaktionsprodukt ab und kristallisiert es aus Äthanol um.

Ausbeute: 2 g (56% der Theorie),

Schmelzpunkt: 150-152 °C

Beispiel 46

3-[4-(2-Benzolsulfonylaminoäthyl)-benzoyl]buttersäure

35,2 g (0,1 Mol) 4-(2-Benzolsulfonylaminoäthyl)-2'-chlorpropiophenon werden mit 18 g (0,11 Mol) Malonsäurediäthylester und 22,4 g (0,2 Mol) Kalium-tert.butanolat in Dimethylsulfoxid zu 2-Carbäthoxy-3-[4-(2-benzolsulfonylaminoäthyl)-benzoyl]buttersäure-äthylester, einem Öl, umgesetzt. Dieses Öl wird durch einstündiges Kochen mit überschüssigem Natriumhydroxid in Äthanol verseift. Nach Eindampfen, Lösen in Wasser und Ansäuern wird daraus die 2-Carboxy-3-[4-(2-benzolsulfonylaminoäthyl)-benzoyl]buttersäure erhalten, welche in Diäthylenglykoldiäthyläther auf 120 °C erhitzt wird. Nach beendeter $CO_2$-Entwicklung wird mit Wasser verdünnt, das Reaktionsprodukt mit Chloroform extrahiert. Das rohe Reaktionsprodukt wird durch Kochen mit methanolischem Chlorwasserstoff in den öligen 3-[4-(2-Benzolsulfonylaminoäthyl)-benzoyl]buttersäuremethylester übergeführt. Dieser Ester wird zur Reinigung an Kieselgel mit Cyclohexan-Essigester = 1:1 chromatographiert und anschließend mit Natriumhydroxid hydrolisiert. Die daraus gewonnene freie Säure wird aus Diisopropyläther umkristallisiert.

Ausbeute: 8 g (20 % der Theorie),

Schmelzpunkt: 85-88 °C

Beispiel 47

[4-(2-Benzolsulfonylaminoäthyl)-benzoyl]-essigsäureäthylester

Eine Suspension von 24,7 g (0,22 Mol) Kalium-tert.butylat in 50 ml Kohlensäurediäthylester wird unter Rühren und unter einem leichten Vakuum auf 80-90 °C erhitzt. Dazu gibt man portionsweise eine Suspension von 29,8 g (0,098 Mol) 4-(2-Benzolsulfonylaminoäthyl)-acetophenon in 200 ml Kohlensäurediäthylester. Das bei der Reaktion entstehende Äthanol destilliert man laufend ab. Nach 5 Stunden bei 80-90 °C destilliert man bei 12 mmHg den gesamten Kohlensäurediäthylester ab, löst den Rückstand in Eiswasser und säuert an. Das Reaktionsprodukt wird mit Äther extrahiert, gewaschen und getrocknet.

Nach dem Eindampfen wird der Rückstand zweimal aus Äthanol umkristallisiert.

Ausbeute: 14,3 g (39 % der Theorie),

Schmelzpunkt: 80-82°C.

Beispiel 48

6-[4-(2-Benzolsulfonylaminoäthyl)-phenyl]pyridazin-(2H)-3-on

Man erhitzt 5,4 g (0,015 Mol) 3-[4-(2-Benzolsulfonylaminoäthyl)-benzoyl]-acrylsäure und 4,1 g 99%iges Hydrazinhydrat eine Stunde lang in 15 ml Eisessig zum Sieden. Man fällt das Reaktionsprodukt mit Wasser aus, extrahiert es mit Chloroform, reinigt es durch Umkristallisieren aus Chloroform/Äthanol und aus n-Butanol.

Ausbeute: 0,6 g (11% der Theorie),

Schmelzpunkt: 168-170°C

$C_{18}H_{17}N_3O_3S$ (355,43)

| | | | | | | | | |
|---|---|---|---|---|---|---|---|---|
| Ber.: | C | 60,83 | H | 4,82 | N | 11,82 | S | 9,02 |
| Gef.: | | 60,87 | | 5,13 | | 11,87 | | 8,76 |

Beispiel 49

4-Hydroxy-4-[4-(2-benzolsulfonylaminoäthyl)-phenyl]-crotonsäure

7,2 g (0,02 Mol) 3-[4-(2-Benzolsulfonylaminoäthyl)-benzoyl]-acrylsäure werden mit 30 ml 1 molarer Natronlauge in Lösung gebracht und mit 0,4 g (0,01 Mol) Natriumborhydrid versetzt. Nach einer Stunde wird angesäuert und das Reaktionsprodukt mit Essigester extrahiert. Die organische Phase wird getrocknet und eingeengt. Der erhaltene Rückstand wird durch Chromatographie über Kieselgel mit Äthylenchlorid/Äthanol = 50:1 gereinigt.

Ausbeute: 3,2 g (44 % der Theorie),

langsam erstarrendes Öl, Rf-Wert: 0,3 (Kieselgel-Polygram-Platte mit Äthylenchlorid/Essigester/Eisessig = 100:30:5).

$C_{18}H_{19}NO_5S$ (361,43)

| | | | | | | | | |
|---|---|---|---|---|---|---|---|---|
| Ber.: | C | 59,82 | H | 5,30 | N | 3,88 | S | 8,87 |
| Gef.: | | 59,61 | | 5,31 | | 3,70 | | 9,10 |

Beispiel 50

3-[4-(2-Benzolsulfonylamino-äthyl)-benzoyl]propionsäure

Man suspendiert 71,2 g (0,534 Mol) wasserfreies Aluminiumchlorid in 250 ml Äthylenchlorid und gibt unter kräftigem Rühren 26,7 g (0,267 Mol) Bernsteinsäureanhydrid und anschließend 46,5 g (0,178 Mol) Benzolsulfonsäure-(N,β-phenyläthyl)-amid zu. Man rührt eine Stunde bei Raumtemperatur, zersetzt mit Eis und 75 ml konzentrierter Salzsäure und treibt das Lösungsmittel mit Wasserdampf ab. Das Produkt kristallisiert aus. Nach Absaugen, Waschen und Trocknen wird es aus 300 ml Äthanol umkristallisiert.

Ausbeute: 42,7 g (66 % der Theorie),

Schmelzpunkt: 152-154°C

$C_{18}H_{19}NO_5S$ (361,43)

| | | | | | | | | |
|---|---|---|---|---|---|---|---|---|
| Ber.: | C | 59,82 | H | 5,30 | N | 3,88 | S | 8,87 |
| Gef.: | | 59,80 | | 5,29 | | 3,94 | | 9,10 |

Beispiel 51

3-[4-(2-Benzolsulfonylamino-äthyl)-benzoyl]propionsäure

7,7 g (0,03 Mol) 3-[4-(2-Aminoäthyl)-benzoyl]-propionsäure-hydrochlorid werden in 25 ml Pyridin suspendiert und mit 100 mg 4-Dimethylaminopyridin und anschließend mit 7 g (0,04 Mol) Benzolsulfonsäurechlorid versetzt. Nach 2 Stunden verdünnt man mit Eiswasser,

säuert an, saugt das Reaktionsprodukt ab und kristallisiert es aus Äthanol um.

Ausbeute: 5,4 g (50 % der Theorie),

Schmelzpunkt: 151-153°C

Beispiel I

Tabletten mit 100 mg 6-[4-(2-Benzoylsulfonylaminoäthyl)-phenyl]-4,5-dihydro-pyridazin-(2H)-3-on

Zusammensetzung:

1 Tablette enthält:

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Massen (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

Tablettengewicht: 220 mg

Durchmesser: 9 mm, biplan mit beidseitiger Facette

und einseitiger Teilkerbe.

Beispiel II

Hartgelatine-Kapseln mit 150 mg 6-[4-(2-Benzoylsulfonylaminoäthyl)-phenyl]-4,5-dihydro-pyridazin-(2H)-3-on

1 Kapsel enthält:

| | | |
|---|---|---|
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 320,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.

Kapselfüllung: ca. 320 mg

Kapselhülle: Hartgelatine-Kapsel Größe 1.

Beispiel III

Suppositorien mit 150 mg 6-[4-(2-Benzoylsulfonylaminoäthyl)-phenyl]-4,5-dihydro-pyridazin-(2H)-3-on

| | |
|---|---|
| 1 Zäpfchen enthält: | |
| Wirkstoff | 150,0 mg |
| Polyäthylenglykol (M.G. 1500) | 550,0 mg |
| Polyäthylenglykol (M.G. 6000) | 460,0 mg |
| Polyäthylensorbitanmonostearat | 840,0 mg |
| | 2 000,0 mg |

Herstellung:

Nach dem Aufschmelzen der Suppositorienmassen wird der Wirkstoff darin homogen verteilt und die Schmelze in vorgekühlte Formen gegossen.

Beispiel IV

Suspensionen mit 50 mg 6-[4-(2-Benzoylsulfonylaminoäthyl)-phenyl]-4,5-dihydro-pyridazin-(2H)-3-on

| | | |
|---|---|---|
| 100 ml Suspension enthalten: | | |
| Wirkstoff | | 1,0 g |
| Carboxymethylcellulose-Na-Salz | | 0,2 g |
| p-Hydroxybenzoesäuremethylester | | 0,05 g |
| p-Hydroxybenzoesäurepropylester | | 0,01 g |
| Glycerin | | 5,0 g |
| Sorbitlösung 70%ig | | 50,0 g |
| Aroma | | 0,3 g |
| Wasser dest. | ad | 100 ml |

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe der Sorbitlösung und des Aromas wird die Suspension zur Entlüftung unter Rühren evakuiert.

5 ml Suspension enthalten 50 mg Wirkstoff.

Beispiel V

Tabletten mit 150 mg 6-[4-(2-Benzoylsulfonylaminoäthyl)-phenyl]-4,5-dihydro-pyridazin-(2H)-3-on

Zusammensetzung:

1 Tablette enthält:

| | |
|---|---|
| Wirksubstanz | 150,0 mg |

| | |
|---|---|
| Milchzucker pulv. | 89,0 mg |
| Maisstärke | 40,0 mg |
| Kolloidale Kieselsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

Tablettengewicht: 300 mg

Stempel: 10 mm, flach

Beispiel VI

Filmtabletten mit 75 mg 6-[4-(2-Benzoylsulfonylaminoäthyl)-phenyl]-4,5-dihydro-pyridazin-(2H)-3-on

1 Tablettenkern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm Maschenweite gerieben und mit der restlichen Menge Magnesiumstearat vermischt. Dieses Granulat wird auf einer Tablettiermaschine zu Tabletten mit der gewünschten Form gepreßt.

Kerngewicht: 230 mg

Stempel: 9 mm, gewölbt

Die so hergestellten Tablettenkerne werden mit einem Film überzogen, der im wesentlichen aus Hydroxypropylmethylcellulose besteht. Die fertigen Filmtabletten werden mit Bienenwachs geglänzt.

Filmtablettengewicht: 245 mg

Selbstverständlich können alle übrigen Verbindungen der allgemeinen Formel I als Wirkstoffe in den vorstehenden galenischen Zubereitungen eingesetzt werden.

**Ansprüche**

1. Neue Sulfonylaminoäthylverbindungen der allgemeinen Formel

$$R_1-SO_2N(R_2)-CH_2-A-B-E \qquad ,(I)$$

in der

$R_1$ eine gegebenenfalls durch eine Methylgruppe oder ein Halogenatom mono-der disubstituierte Phenylgruppe, wobei die Substituenten gleich oder verschieden sein können, eine gegebenenfalls durch ein Halogenatom substituierte Biphenylylgruppe, eine Naphthyl-, Pyridyl-oder Thienylgruppe,

$R_2$ ein Wasserstoffatom oder eine Methylgruppe,

A eine Methylen-oder Methylenoxygruppe, wobei das Sauerstoffatom mit dem benachbarten Rest B verknüpft ist,

B eine Phenylen-oder Naphthylengruppe und

E eine über eine geradkettige oder verzweigte Alkylengruppe mit 2 bis 5 Kohlenstoffatomen oder über eine Alkenylengruppe mit 3 bis 5 Kohlenstoffatomen gebundene Hydroxycarbonyl-oder Alkoxycarbonylgruppe, wobei jeweils eine Methylengruppe der vorstehend erwähnten Alkylen-oder Alkenylenreste, welche mit dem Rest B verknüpft sein muß, durch eine Hydroxymethylen-oder Carbonylgruppe ersetzt ist und der Alkoxyteil, welcher in 2-oder 3-Stellung durch eine Alkoxygruppe mit 1 bis 3 Kohlenstoffatomen substituiert sein kann, 1 bis 3 Kohlenstoffatome enthalten kann, oder eine gegebenenfalls im Kohlenstoffgerüst durch 1 oder 2 Alkylgruppen mit insgesamt 1 bis 3 Kohlenstoffatomen substituierte 4,5-Dihydro-pyridazin-3-on-6-yl-oder Pyridazin-3-on-6-yl-gruppe bedeuten, und deren Salze mit anorganischen oder organischen Basen, falls E eine Hydroxycarbonylgruppe enthält oder darstellt.

2. Neue Sulfonylaminoäthylverbindungen der allgemeinen Formel I gemäß Anspruch 1, in der

$R_1$ eine gegebenenfalls durch eine Methylgruppe, durch ein Fluor-, Chlor-oder Bromatom substituierte Phenylgruppe, eine gegebenenfalls durch ein Fluor-oder Chloratom substituierte 4-Biphenylgruppe, eine Naphthyl-, Pyridyl-oder Thienylgruppe,

$R_2$ ein Wasserstoffatom oder eine Methylgruppe,

A eine Methylen-oder Methylenoxygruppe, wobei das Sauerstoffatom mit dem benachbarten Rest B verknüpft ist,

B eine 1,4-Phenylen-oder 1,4-Naphthylengruppe und

E eine über eine geradkettige oder verzweigte Alkylengruppe mit 2 bis 4 Kohlenstoffatomen oder über eine geradkettige oder verzweigte Alkenylengruppe mit 3 oder 4 Kohlenstoffatomen gebundene Hydroxycarbonyl-oder Alkoxycarbonylgruppe mit insgesamt 2 oder 3 Kohlenstoffatomen, wobei jeweils eine Methylengruppe der vorstehend erwähnten Alkylen-oder Alkenylengruppen welche mit dem Rest B verknüpft sein muß, durch eine Hydroxymethylen-oder Carbonylgruppe ersetzt ist und eine Äthoxycarbonylgruppe in 2-Stellung durch eine Methoxygruppe substituiert sein kann, wobei jedoch E keine gegebenenfalls in α-Stellung durch eine Methyl-oder Äthylgruppe substituierte Hydroxycarbonylmethylencarbonylgruppe darstellen kann, oder eine gegebenenfalls in 5-Stellung durch eine Methylgruppe substituierte 4,5-Dihydro-pyridazin-3-on-6-yl-oder Pyridazin-3-on-6-yl-gruppe bedeuten, und deren Salze mit anorganischen oder organischen Basen, falls E eine Hydroxycarbonylgruppe enthält oder darstellt.

3. Neue Sulfonylaminoäthylverbindungen der allgemeinen Formel I gemäß Anspruch 1, in der

$R_1$ eine gegebenenfalls durch ein Chloratom oder eine Methylgruppe substituierte Phenylgruppe,

$R_2$ ein Wasserstoffatom,

A eine Methylen-oder Methylenoxygruppe,

B eine 1,4-Phenylengruppe und

E eine über eine n-Propylengruppe gebundene Hydroxycarbonyl-oder Alkoxycarbonylgruppe mit insgesamt 2 oder 3 Kohlenstoffatomen, wobei jeweils eine Methylengruppe der vorstehend erwähnten Propylengruppe, welche mit dem Rest B verknüpft sein muß, durch eine Hydroxymethylen-oder Carbonylgruppe ersetzt ist und eine Äthoxycarbonylgruppe in 2-Stellung durch eine Methoxygruppe substituiert sein kann, oder eine gegebenenfalls in 5-Stellung durch eine Methylgruppe substituierte 4,5-Dihydro-pyridazin-3-on-6-yl-gruppe bedeuten, und deren Salze mit anorganischen oder organischen Basen, falls E eine Hydroxycarbonylgruppe enthält oder darstellt.

4. 3-[4-(2-Benzolsulfonylaminoäthyl)-benzoyl]propionsäure und deren Additionssalze mit anorganischen Basen.

5. 6-[4-(2-Benzoylsulfonylaminoäthyl)-phenyl]-4,5-dihydro-pyridazin-(2H)-3-on.

6. Physiologisch verträgliche Additionssalze der Verbindungen gemäß den Ansprüchen 1 bis 4 mit anorganischen oder organischen Basen.

7. Arzneimittel, enthaltend als Wirkstoff eine Verbindung gemäß den Ansprüchen 1 bis 5 oder dessen physiologisch verträgliches Additionssalz gemäß Anspruch 6 neben gegebenenfalls einem oder mehreren inerten Trägerstoffen und/oder Verdünnungsmitteln.

8. Verwendung einer Verbindung gemäß den Ansprüchen 1 bis 5 oder dessen physiologisch verträgliches Additionssalz gemäß Anspruch 6 zur Herstellung eines Arzneimittels zur Behandlung und zur Prophylaxe thrombo-embolischer Erkrankungen, zur Prophylaxe der Arteriosklerose und zur Metastasenprophylaxe.

9. Verfahren zur Herstellung eines Arzneimittels gemäß

Anspruch 7, dadurch gekennzeichnet, daß auf nicht-chemischem Wege eine Verbindung gemäß den Ansprüchen 1 bis 5 oder dessen physiologisch verträgliches Additionssalz gemäß Anspruch 6 in einen oder mehrere inerte übliche Trägerstoffe und/oder Verdünnungsmittel eingearbeitet wird.

10. Verfahren zur Herstellung der Verbindungen gemäß den Ansprüchen 1 bis 6, dadurch gekennzeichnet, daß

a.) eine Verbindung der allgemeinen Formel

$$H - \overset{R_2}{\overset{|}{N}} - CH_2 - A - B - E \qquad ,(II)$$

in der

$R_2$, A, B und E wie in den Ansprüchen 1 bis 5 definiert sind, wobei eine Hydroxygruppe im Rest E durch eine hydrolytisch oder hydrogenolytisch abspaltbare Schutzgruppe geschützt sein kann, mit einem Phenylsulfonsäurederivat der allgemeinen Formel

$R_1$ $-SO_2X$ ,(III)

in der

$R_1$ wie in den Ansprüchen 1 bis 5 definiert ist und X eine nucleophile Austrittsgruppe darstellt, umgesetzt und gegebenenfalls anschließend ein verwendeter Schutzrest abgespalten wird oder

b.) zur Herstellung von Verbindungen der allgemeinen Formel I, in der E eine über eine Alkylen-oder Alkenylengruppe gebundene Hydroxycarbonylgruppe darstellt, ein Schutzrest von einer Verbindung der allgemeinen Formel

$$R_1-SO_2\overset{R_2}{\overset{|}{N}}-CH_2-A-B-E_1 \qquad ,(IV)$$

in der

$R_1$, $R_2$, A und B wie in den Ansprüchen 1 bis 5 definiert sind und

$E_1$ die für E in den Ansprüchen 1 bis 5 erwähnten Bedeutungen besitzt, wobei jedoch die Carboxygruppe durch einen hydrolytisch, thermolytisch oder hydrogenolytisch abspaltbare Schutzgruppe geschützt ist oder ein funktionelles Derivat der Carboxygruppe darstellt und/oder der Rest $E_1$ eine durch einen Schutzrest geschützte Hydroxygruppe enthält, hydrolytisch, hydrogenolytisch oder thermolytisch abgespalten wird oder

c.) zur Herstellung von Verbindungen der allgemeinen Formel I, in der E benachbart zum Rest B eine Carbonylgruppe enthält, eine Verbindung der allgemeinen Formel

$$R_1-SO_2\overset{R_2}{\overset{|}{N}}-CH_2-A-B-H \qquad ,(V)$$

in der

$R_1$, $R_2$, A und B wie in den Ansprüchen 1 bis 5 definiert sind, mit einer Verbindung der allgemeinen Formel

Y $-E_2$ ,(VI)

in der

$E_2$ die für E in den Ansprüchen 1 bis 5 erwähnten Bedeutungen besitzt, wobei jedoch E benachbart zu Y eine Carbonylgruppe enthalten muß und gleichzeitig eine gegebenenfalls vorhandene Hydroxycarbonylgruppe durch einen hydrolytisch oder hydrogenolytisch abspaltbaren Schutzrest geschützt sein kann, und

Y eine nucleophile Austrittsgruppe darstellen, oder dessen Anhydrid in Gegenwart einer Lewis-Säure umgesetzt und gegebenenfalls anschließend ein verwendeter Schutzrest abgespalten wird oder

d.) zur Herstellung von Verbindungen der allgemeinen Formel I, in der E benachbart zum Rest B eine Hydroxymethylengruppe enthält, eine Verbindung der allgemeinen Formel

$$\begin{array}{l} \quad\; R_2 \\ \quad\;\; | \\ R_1-SO_2N-CH_2-A-B-E_3 \end{array} \qquad ,(VII)$$

in der

$R_1$, $R_2$, A und B wie in den Ansprüchen 1 bis 5 definiert sind und

$E_3$ die für E in den Ansprüchen 1 bis 5 erwähnten Bedeutungen aufweist, wobei jedoch E eine Carbonylgruppe enthalten muß, reduziert wird oder

e.) zur Herstellung von Verbindungen der allgemeinen Formel I, in der E einen der in den Ansprüchen 1 bis 5 erwähnten gesättigten Reste darstellt, eine Verbindung der allgemeinen Formel

$$\begin{array}{l} \quad\; R_2 \\ \quad\;\; | \\ R_1-SO_2N-CH_2-A-B-E_4 \end{array} \qquad ,(VIII)$$

in der

$R_1$, $R_2$, A und B wie in den Ansprüchen 1 bis 5 definiert sind und

$E_4$ einen der für E in den Ansprüchen 1 bis 5 erwähnten ungesättigen Rest darstellt, hydriert wird oder

f.) zur Herstellung von Verbindungen der allgemeinen Formel I, in der E eine der in den Ansprüchen 1 bis 5 erwähnten über eine gesättigte Alkylengruppe gebundene Hydroxycarbonylgruppe darstellt, eine gegebenenfalls im Reaktionsgemisch hergestellte Verbindung der allgemeinen Formel

$$\begin{array}{l} \quad\; R_2 \\ \quad\;\; | \\ R_1-SO_2N-CH_2-A-B-E_5 \end{array} \qquad ,(IX)$$

in der

$R_1$, $R_2$, A und B wie in den Ansprüchen 1 bis 5 definiert sind und

$E_5$ am gleichen Kohlenstoffatom zwei über eine geradkettige oder verzweigte Alkylengruppe mit 1 bis 5 Kohlenstoffatomen gebundene Hydroxycarbonylgruppen darstellt, wobei jeweils eine Methylengruppe des Restes $E_5$, welche mit dem Rest B verknüpft sein muß, durch eine Hydroxymethylen- oder Carbonylgruppe ersetzt ist, decarboxyliert wird oder

g.) zur Herstellung von Verbindungen der allgemeinen Formel I, in der E einen Pyridazinonring darstellt, eine Verbindung der allgemeinen Formel

$$\begin{array}{l} \quad\; R_2 \qquad\qquad\qquad\qquad\qquad\; W \quad R_6 \;\; W \\ \quad\;\; | \qquad\qquad\qquad\qquad\qquad\quad\;\; | \quad\;\; | \quad\;\; | \\ R_1-SO_2N-CH_2-A-B-CO-CH-\!\!\!-\!\!\!-CH-COOH \\ \qquad\qquad\qquad\qquad\qquad\qquad\qquad\quad\;\;\; | \\ \qquad\qquad\qquad\qquad\qquad\qquad\qquad\quad\; R_7 \end{array} \qquad ,(X)$$

in der

$R_1$, $R_2$, A und B wie in den Ansprüchen 1 bis 5 definiert sind,

$R_6$ und $R_7$, die gleich oder verschieden sein können, Wasserstoffatome oder Alkylgruppen, wobei $R_6$ und $R_7$ zusammen insgesamt 1 bis 3 Kohlenstoffatome enthalten können, und W jeweils ein Wasserstoffatom oder zusammen eine weitere Bindung darstellen, oder deren reaktionsfähige Derivate mit Hydrazin umgesetzt wird oder

h) zur Herstellung von Verbindungen der allgemeinen Formel I, in der A eine Methylenoxygruppe darstellt, eine Verbindung der allgemeinen Formel

$$\begin{array}{c} R_2 \\ | \\ R_1\text{-}SO_2\text{-}N\text{-}CH_2\text{-}CH_2\text{-}Z \end{array} \quad ,(XI)$$

in der

$R_1$ und $R_2$ wie in den Ansprüchen 1 bis 5 definiert sind und

Z eine nucleophile Austrittsgruppe wie ein Halogenatom oder eine Sulfonyloxygruppe bedeutet, mit einer Verbindung der allgemeinen Formel

HO -B -E ,(XII)

in der

B und E wie in den Ansprüchen 1 bis 5 definiert sind, wobei eine im Rest B gegebenenfalls vorhandene Hydroxygruppe durch eine hydrolytisch abspaltbare Schutzgruppe wie eine Alkoxy-oder Benzyloxygruppe geschützt sein kann, gegebenenfalls anschließend ein verwendeter Schutzrest und gegebenenfalls anschließend ein erhaltener Ester der allgemeinen Formel I in die entsprechende Carbonsäure übergeführt wird oder

i.) zur Herstellung von Verbindungen der allgemeinen Formel I, in der E eine gegebenenfalls in $\alpha$-Stellung durch eine Methyl-oder Äthylgruppe substituierte Alkoxycarbonylmethylencarbonylgruppe darstellt, ein Acetophenon der allgemeinen Formel

$$\begin{array}{c} R_2 \\ | \\ R_1\text{-}SO_2N\text{-}CH_2\text{-}A\text{-}B\text{-}CO\text{-}CH_2\text{-}R_8 \end{array} \quad ,(XIII)$$

in der

$R_1$, $R_2$, A und B wie in den Ansprüchen 1 bis 5 definiert sind und

$R_8$ ein Wasserstoffatom, eine Methyl-oder Äthylgruppe darstellt, mit einem Kohlensäuredialkylester in Gegenwart einer Base umgesetzt wird und

gewünschtenfalls anschließend eine so erhaltene Verbindung der allgemeinen Formel I, falls diese eine Carboxygruppe enthält, in ihre Additionssalze mit anorganischen oder organischen Basen, insbesondere für die pharmazeutische Anwendung in ihre physiologisch verträglichen Additionssalze, übergeführt wird.